# FASCICULE DE BREVET EUROPEEN

(11) **EP 1 478 623 B1**
(45) Date de publication et mention de la délivrance du brevet: **23.03.2011**
(21) Numéro de dépôt: 03718910.7
(22) Date de dépôt: 26.02.2003
(51) Int. Cl.: C07D 207/44, G01N 33/53

(54) **NOUVEAUX AGENTS DE COUPLAGES, LEURS INTERMEDIAIRES ACTIVES, LEURS CONJUGUES, AINSI QUE L'UTILISATION DE CES CONJUGUES DANS DES METHODES DE DIAGNOSTIC**
KUPPLUNGSMITTEL, DEREN AKTIVE ZWISCHENVERBINDUNGEN UND KONJUGATE SOWIE DIE VERWENDUNG DIESER KONJUGATE FÜR DIAGNOSTISCHE ZWECKE
NOVEL COUPLING AGENTS, THE ACTIVE INTERMEDIATES AND THE CONJUGATES THEREOF AND THE USE OF SAID CONJUGATES IN DIAGNOSTIC METHODS

(30) Priorité: 28.02.2002 FR 0202559
(43) Date de publication de la demande: 24.11.2004
(73) Titulaire: BIOMERIEUX, 69280 Marcy-L'Etoile (FR)
(72) Inventeur: LACOUX, Xavier, F-69380 Dommartin (FR)
(74) Mandataire: Sarlin, Laure V.
(86) Numéro de dépôt international: PCT/FR2003/000619
(87) Numéro de publication internationale: WO 2003/072546

(56) Documents cités:
- WO-A-99/13919
- US-A- 5 162 505
- US-A- 5 929 211
- MIKOLAJCZYK, STEPHEN D. ET AL: "High Yield, Site-Specific Coupling of N-Terminally Modified.beta.-Lactamase to a Proteolytically Derived Single-Sulfhydryl Murine Fab'" BIOCONJUGATE CHEMISTRY (1994), 5(6), 636-46, XP002220206 cité dans la demande

## Description

La présente invention concerne des agents de couplage hétérobifonctionnels utiles notamment pour le couplage de molécules présentant à leur surface au moins une fonction aldéhyde et/ou cétone et de molécules présentant à leur surface au moins une fonction thiol libre.

Les agents de couplage présentent une utilité dans le domaine du diagnostic, notamment dans la détection de réactions du type ligand-anti-ligand tel que antigèneanticorps ou protéine-protéine, car ils permettent de coupler directement une molécule d'intérêt biologique, telle qu'un antigène, à une molécule de révélation, telle qu'une enzyme. La liaison de la molécule biologique d'intérêt à une autre molécule, telle qu'un anticorps, est mise en évidence grâce à la molécule de révélation.

Pour ce faire, les agents de couplage doivent être bifonctionnels et présenter à la fois une fonction chimique permettant leur couplage avec ladite molécule d'intérêt biologique et une autre fonction chimique permettant leur couplage avec ladite molécule de révélation, ainsi qu'un bras espaceur permettant d'éloigner suffisamment les molécules couplées.

On connaît de l'art antérieur différents agents de couplage hétérobifonctionnels. Ces agents de couplage, que l'on trouve par exemple dans le catalogue PIERCE, présentent essentiellement une fonction maléimide, une fonction hydrazone et un bras espaceur du type alkyle saturé ou encore aromatique. Ces agents de couplage sont utilisés pour le couplage, d'une part de molécules présentant des fonctions thiol libres et d'autre part de molécules présentant des fonctions aldéhyde ou cétone. Toutefois, ces agents de couplage ont pour inconvénient qu'ils sont peu solubles dans les milieux aqueux du fait notamment du caractère hydrophobe du bras espaceur, et nécessitent souvent l'utilisation de co-solvants organiques lors de leur utilisation, notamment lors de la préparation des conjugués. De plus, les protéines couplées à ces agents de couplage ont tendance à précipiter au cours du marquage.

Mikolajczyk S. et al. (Bioconjugate Chemistry, 1994, 5(6), 636-646) décrivent l'utilisation d'un agent de couplage présentant une fonction maléimide, une fonction aminooxyalkylène et un bras espaceur relativement court contenant notamment la L-lysine. Cet agent de couplage est utilisé pour le couplage de deux protéines modifiées que sont la β-lactamase et le fragment peptidique murin Fab. Cet agent de couplage a pour inconvénients qu'il possède un bras espaceur court ne permettant pas d'éloigner suffisamment les molécules couplées à l'agent de couplage, et que son procédé de synthèse est long. Par ailleurs, il pourrait se poser un problème de stabilité de l'agent de couplage du fait de la présence de la fonction -COOH de la lysine qui pourrait réagir avec la fonction alcoxyamine qui est connue comme étant très réactive.

La demanderesse a maintenant mis au point de nouveaux agents de couplage permettant de pallier les inconvénients des agents de couplage de l'art antérieur en ce sens qu'ils permettent d'éloigner suffisamment les molécules couplées, qu'ils sont très solubles du fait de la nature de leur bras espaceur et que les conjugués obtenus sont très stables. De plus, les agents de couplage de l'invention permettent d'améliorer la sensibilité de détection dans des tests diagnostics.

Ainsi, un premier objet de la présente invention consiste en des agents de couplage de formule générale (I) suivante : 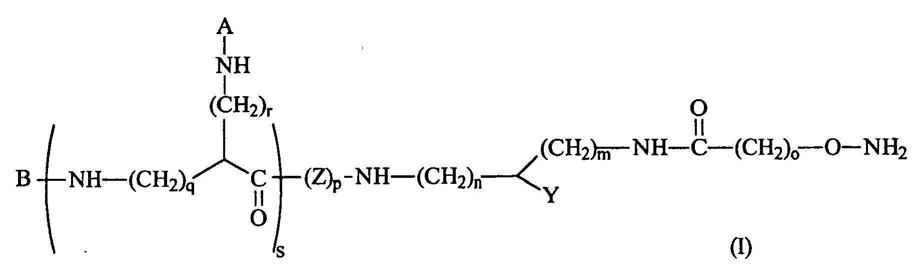 dans laquelle :
- Z représente -NH-(CH₂)₂-O-(CH₂)₂-O-CH₂-C(O)-,
- A représente un groupe choisi parmi : 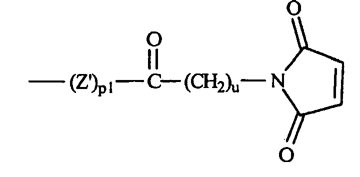 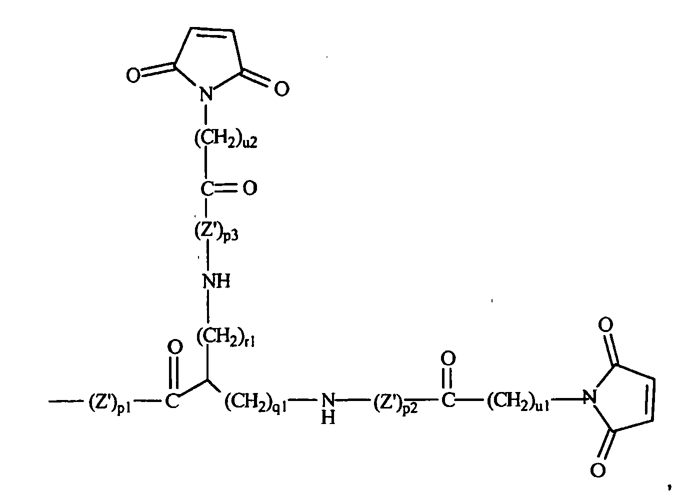 et 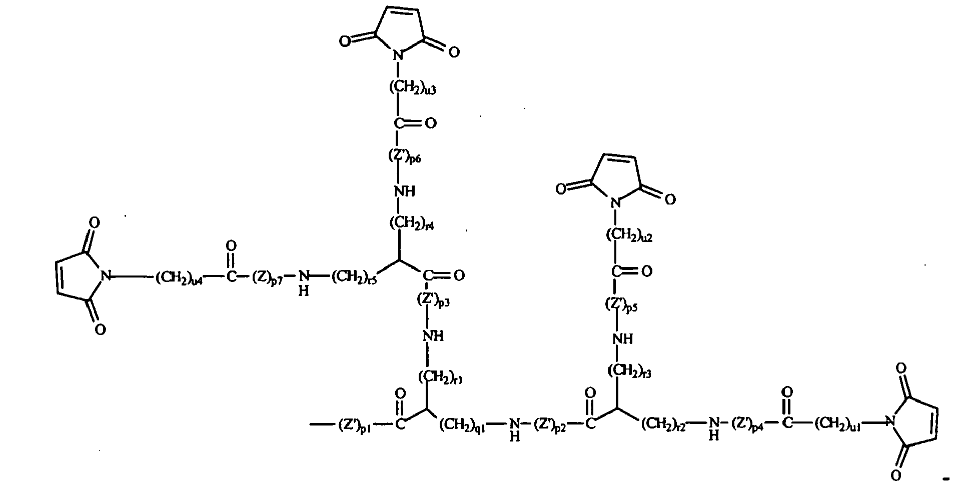
B représente un groupe choisi parmi : 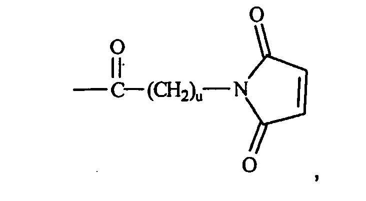 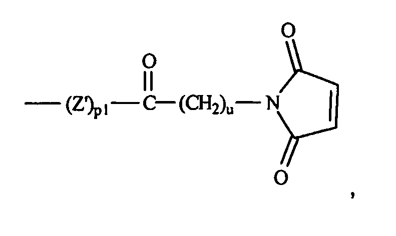 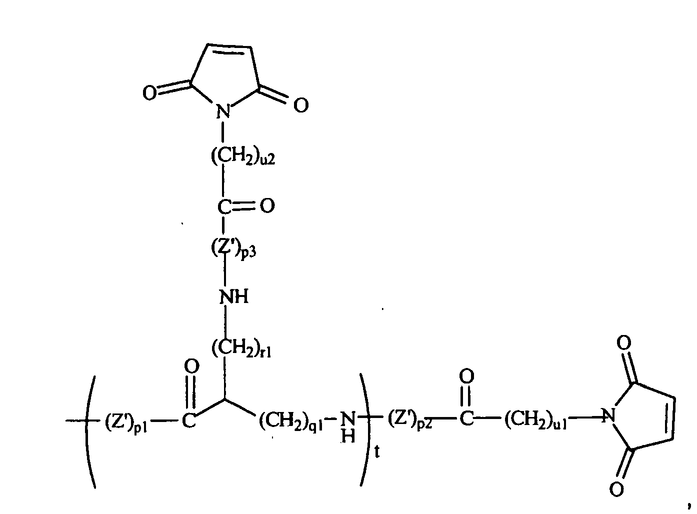 et 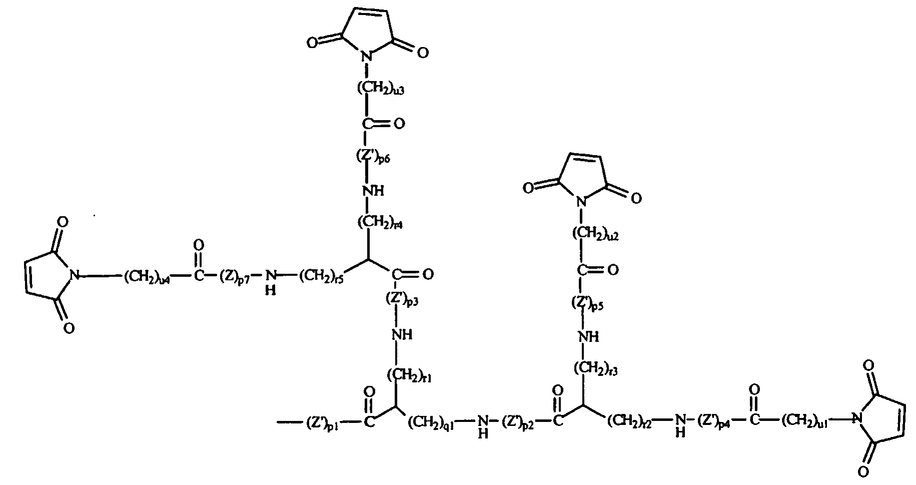
   - Y représente un groupement qui se termine par -C(O)-NH₂ et qui est inerte vis à vis de la fonction aminooxyalkylène, du ou des groupes maléimide de B et éventuellement de A et du motif -(CH₂)₂-O-(CH₂)₂-O-CH₂- de Z,
   - Z' représente -C(O)-CH₂-O-(CH₂)₂-O-(CH₂)₂-NH-,
   - n est un nombre entier compris entre 0 et 3,
   - m est un nombre entier compris entre 1 et 3,
   - o est un nombre entier compris entre 1 et 3,
   - p et p1 à p7 sont chacun indépendamment un nombre entier compris entre 1 et 4,
   - q et q1 sont chacun indépendamment un nombre entier compris entre 0 et 3,
   - r et r1 à r5 sont chacun indépendamment un nombre entier compris entre 2 et 5,
   - s est un nombre entier égal à 0 ou 1,
   - t est un nombre entier compris entre 1 et 7, et
   - u et u1 à u4 sont chacun indépendamment un nombre entier compris entre 2 et 10.

Un autre objet de l'invention consiste en des intermédiaires activés constitués d'un agent de couplage tel que défini ci-dessus et de :
- une molécule présentant à sa surface au moins une fonction aldéhyde et/ou cétone avant conjugaison, ou
- une à huit molécules présentant en surface au moins une fonction thiol libre avant conjugaison.

Un autre objet de l'invention consiste en des conjugués constitués d'un agent de couplage tel que défini ci-dessus lié d'une part à une molécule présentant à sa surface au moins une fonction aldéhyde et/ou cétone avant conjugaison, qui est de préférence une molécule d'intérêt biologique, et d'autre part à une à huit molécules présentant en surface au moins une fonction thiol libre avant conjugaison, qui sont de préférence des marqueurs.

Enfin, un dernier objet de l'invention consiste en l'utilisation d'un conjugué tel que défini ci-dessus dans des méthodes de diagnostic *in vitro* de maladies impliquant une reconnaissance d'un couple ligand-anti-ligand.

Les agents de couplage de l'invention sont tels que définis dans la formule (I) ci-dessus. Ils sont caractérisés par la présence de :
- une fonction aminooxyalkylène : NH₂-O-(CH₂)ₒ-, o étant tel que défini pour (I), pour pouvoir se lier spécifiquement avec une molécule présentant à sa surface au moins une fonction aldéhyde et/ou cétone,
- une à huit fonctions maléimide au niveau de B et éventuellement de A pour pouvoir se lier spécifiquement avec une à huit molécules présentant en surface au moins une fonction thiol libre et
- un bras espaceur Z du type polyéthylèneglycol qui est long et qui permet de conférer une meilleure solubilité à l'agent de couplage.

De plus, l'association des trois caractéristiques ci-dessus permet de conférer une meilleure stabilité aux conjugués formés à partir desdits agents de couplage.

Les agents de couplage de l'invention contiennent également un groupement Y inerte vis à vis de la fonction aminooxyalkylène : NH₂-O-(CH₂)ₒ-, o étant tel que défini pour (I), du ou des groupes maléimide de B et éventuellement de A et du motif-(CH₂)₂-O-(CH₂)₂-O-CH₂- de Z, c'est à dire que le groupement Y n'est pas susceptible de réagir avec ces motifs ou fonctions. De plus, Y se termine par -C(O)-NH₂. Cette terminaison - C(O)-NH₂ est importante car, contrairement à la fonction -COOH telle que présente dans l'agent de couplage décrit dans Bioconjugate chemistry, 1994 ,556, 636-646, elle permet d'éviter l'éventuel problème de stabilité dû à la présence de l'aminooxyalkylène. De plus, l'agent de couplage ainsi obtenu ne présente pas de charge ionique. Selon un mode de réalisation préféré, Y est -C(O)-NH₂.

Les agents de couplage de l'invention peuvent comporter de une à huit fonctions maléimide permettant la fixation de une à huit molécules présentant en surface au moins une, en général plusieurs fonctions thiol libres avant conjugaison, respectivement.

On notera que « n est un nombre entier compris entre 0 et 3 » par exemple, signifie que n peut être égal à 0, 1, 2 ou 3.

Dans l'agent de couplage (I), chaque groupe Z ou Z' est lié par sa fonction -NH- à une fonction -C(O)- et par sa fonction -C(O)- à une fonction -NH-.

Les agents de couplage selon l'invention comporte au moins un carbone asymétrique et les différents isomères optiques font partie intégrante de l'invention. La présente invention a donc pour objet les agents de couplage de formule (I) sous forme d'isomères purs mais également sous forme de mélange racémique ou en proportion quelconque. Les agents de couplage (I) sont isolés sous forme d'isomère pur selon les techniques classiques de séparation, par exemple de chromatographie sur phase chirale.

Selon un mode de réalisation préféré, les agents de couplage de l'invention ne possède qu'une seule fonction maléimide. Dans ce cas, s dans la formule (I) est égal à 0 et de préférence B représente : 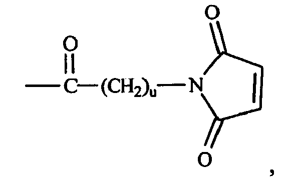 u étant tel que défini précédemment.

Lorsque les agents de couplage de l'invention possèdent plus d'une fonction maléimide, ils peuvent être soit sous forme dendrimère (ou ramifié), soit sous forme linéaire, selon la valeur de A et de B.

Selon un mode de réalisation préféré, A et B sont identiques et représentent : 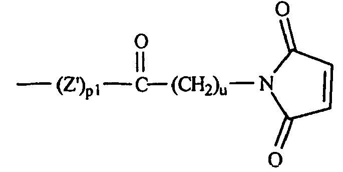 Z', p1 et u étant tels que définis précédemment,
de sorte que les agents de couplage de l'invention sont sous forme dendrimère et possèdent deux fonctions maléimide.

Selon un autre mode de réalisation préféré, A et B sont identiques et représentent : 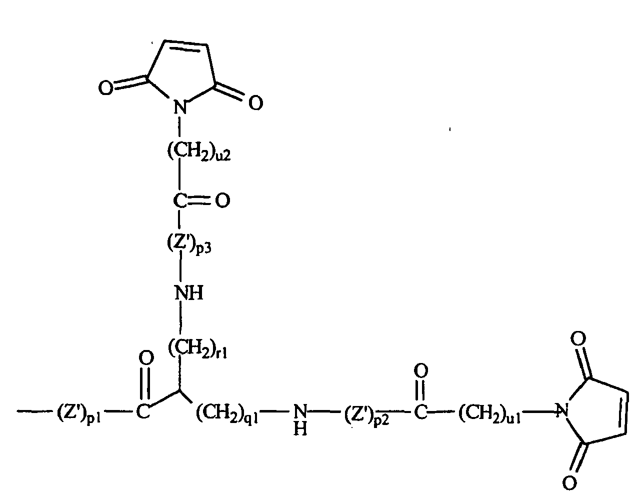 Z', p1, p2, p3, q1, r1, u1 et u2 étant tels que définis précédemment,
de sorte que les agents de couplage de l'invention sont sous forme dendrimère et possèdent quatre fonctions maléimide.

Selon encore un autre mode de réalisation, A et B sont identiques et représentent: 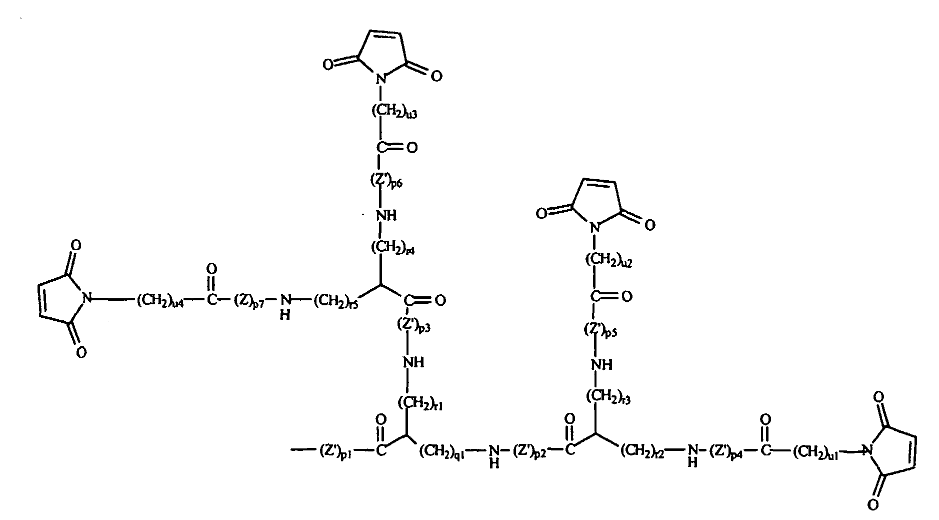 Z, Z', p1 à p7, q1, r1 à r5 et u1 à u4 étant tels que définis précédemment,
de sorte que les agents de couplage de l'invention sont sous forme dendrimère et possèdent huit fonctions maléimide.

Lorsque les agents de couplage sont sous forme linéaire, A représente : 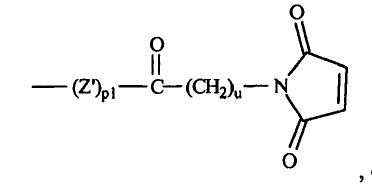 , et
B représente : 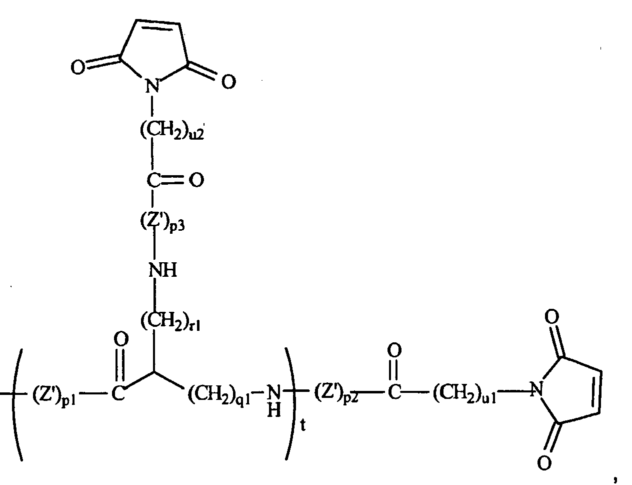 Z', t, p1 à p3, q1, r1 et u, u1 et u2 étant tels que définis précédemment, et ceci constitue un autre mode de réalisation de l'invention.

Dans les agents de couplage de l'invention, le bras de couplage peut avoir une longueur qui varie, mais elle doit être suffisante pour éloigner les molécules qui seront couplées, en particulier du fait de leur encombrement stérique, et lorsque les agents de couplage possèdent plusieurs fonctions maléimide qui se coupleront à autant de molécules à fonctions thiol libres.

La longueur des bras varie en fonction des indices m, n, o, p, p1 à p7, r, r1 à r5, q, q1 et u, u1 à u4.

Selon un mode de réalisation, les agents de couplage de l'invention remplissent au moins l'une des conditions suivantes:
- n est égal à 0,
- m est égal à 1,
- p et p1 à p7, le cas échéant, sont égaux à 2,
- q et q1, le cas échéant, sont égaux à 0,
- r et r1 à r5, le cas échéant, sont égaux à 4,
- u et u1 à u4, le cas échéant, sont égaux à 2 et
- o est égal à 1.

Les agents de couplage préférés sont choisis parmi les suivants :
- composé de formule (I) dans laquelle m = 1, Y = -C(O)-NH₂, o=1, n=0, p=2, s=0 et B représente : 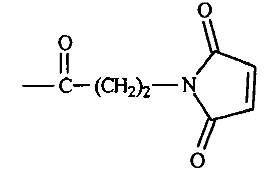 et
- composé de formule (I) dans laquelle m = 1, Y = -C(O)-NH₂, o = 1, n = 0, p = 2, r = 4, s=1, q=0 et A et B sont identiques et représentent : 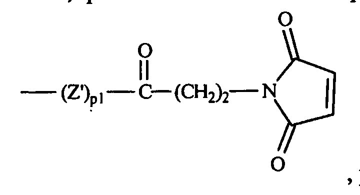 p1 étant égal à 2.

Les agents de couplage de l'invention sont obtenus par synthèse en phase solide de type synthèse peptidique de façon manuelle ou bien, de préférence de façon automatisée sur les synthétiseurs du commerce tels que le synthétiseur ABI 431 A.
En d'autres termes, les agents de couplage, qu'on peut découper en les synthons tels que décrits ci-après, qui sont des synthons d'acide aminé ou assimilé, sont obtenus à partir d'un support de synthèse (phase solide), tel que la résine commerciale RINK-Amide-MBHA (NOVABIOCHEM), qui permet d'attacher l'extrémité C-terminale du premier synthon (synthon I) de l'agent de couplage à synthétiser avant de procéder à la synthèse dudit agent en ajoutant au fur et à mesure les synthons appropriés choisis parmi les synthons II à IV : 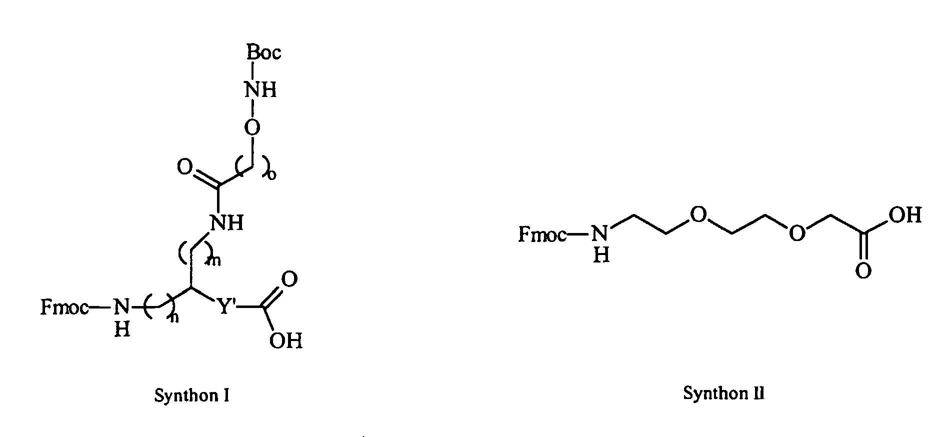 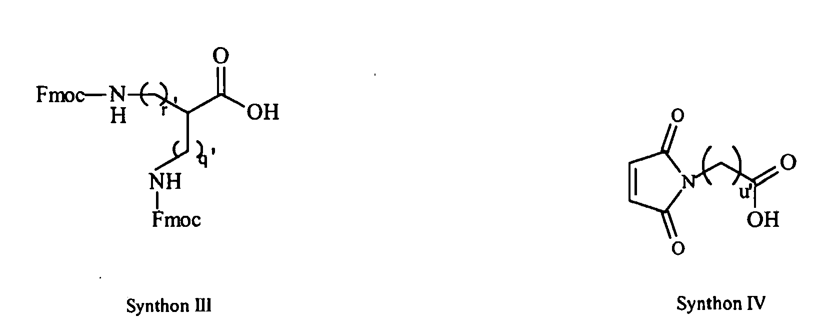 où n, m, et o sont tels que définis précédemment, q'représente q ou q1, r' représente r, r1, r2, r3, r4, ou r5 et u' représente u, u1, u2, u3, ou u4 tels que définis précédemment, Boc signifie *tert*-butyloxycarbonyle, Fmoc signifie fluorénylméthoxycarbonyle et Y' correspond à Y dénué de la fonction -C(O)-NH₂.
Le couplage de d'un synthon IV avec un synthon I se fait après déprotection du groupe Fmoc.

Les réactifs utilisés lors des cycles de couplage des différents synthons sont connus de l'homme du métier et sont décrits par exemple dans Chemical Approaches to the Synthesis of Peptides & Proteins, Paul Lloyd Williams, Fernando Albericio, Ernest Giralt, CRC Press. Après synthèse de l'agent de couplage de l'invention, ce dernier est détaché du support selon un mode opératoire connu de l'homme du métier (Chemical Approaches to the Synthesis of Peptides & Proteins, *supra)* et est déprotégé simultanément (groupement Boc) en présence d'une solution d'acidolyse telle qu'une solution à base d'acide trifluoroacétique.

Les agents de couplage sont ensuite purifiés, par exemple par HPLC semi-préparative BECKMAN sur une colonne en phase inverse C₁₈.

Les agents de couplage ainsi obtenus sont utiles pour le couplage, d'une part, de molécules présentant à leur surface au moins une fonction aldéhyde et/ou cétone avant conjugaison, qui vont réagir avec la fonction aminooxyalkylène des agents, et d'autre part de molécules présentant à leur surface au moins une fonction thiol libre avant conjugaison, qui vont réagir avec la ou les fonctions maléimide desdits agents.

L'invention a donc pour objet des intermédiaires activés constitués d'un agent de couplage de l'invention et soit d'une molécule présentant à sa surface au moins une fonction aldéhyde ou cétone avant conjugaison, soit de une à huit molécules présentant à leur surface au moins une fonction thiol libre avant conjugaison, de préférence de une à quatre desdites molécules.

Selon un mode de réalisation préféré, les intermédiaires activés sont constitués d'un agent de couplage de l'invention et d'une molécule présentant à sa surface au moins une, en général des fonctions aldéhyde et/ou cétone avant conjugaison.

Les molécules présentant à leur surface des fonctions aldéhyde ou cétone comprennent toutes molécules présentant de telles fonctions ou bien modifiées pour inclure de telles fonctions. A titre d'exemple, on peut citer les peptides présentant une sérine ou une thréonine N-terminale, ainsi que les molécules d'intérêt biologique telles que les antigènes, les anticorps, les haptènes, les glycoprotéines, ou toute autre molécule susceptible de se lier à un partenaire de liaison.

La modification des molécules dont on veut qu'elles présentent des fonctions aldéhyde ou cétone peut être effectuée par réaction avec des réactifs amenant de telles fonctions tels que le périodate.

A titre d'exemples de molécules présentant des fonctions aldéhyde, on peut citer les glycoprotéines oxydée au périodate, telle que la glycoprotéine d'enveloppe du virus VIH-1 gp160, les sucres. A titre d'exemples de molécules présentant des fonctions cétone, on peut citer toute molécule qui inclut un groupement lévulinyle (cétone).

Selon un mode de réalisation préféré, la molécule présentant à sa surface des fonctions aldéhyde est une glycoprotéine oxydée au périodate et de préférence la gp160 ainsi oxydée.

L'homme du métier pourra facilement déterminer quelles sont les molécules présentant en surface des fonctions thiol libres, naturellement ou par réaction chimique, avant conjugaison.

Les exemples de telles molécules comprennent les marqueurs, lesquels constituent un mode de réalisation de l'invention.

On entend par marqueur toute molécule capable de générer directement ou indirectement un signal détectable. Une liste non limitative de ces marqueurs consiste en :
- les enzymes qui produisent un signal détectable par exemple par colorimétrie, fluorescence, luminescence, comme la péroxydase de raifort, la phosphatase alcaline, la α-galactosidase, la glucose-6-phosphate déshydrogénase,
- les chromophores comme les composés fluorescents, luminescents, colorants,
- les molécules radioactives contenant du ³²P, du ³⁵S ou de l'¹²⁵I, et
- les molécules fluorescentes,
   étant entendu que ces marqueurs seront modifiés le cas échéant pour présenter des fonctions thiol libres.

Selon un mode de réalisation de l'invention, le marqueur est la phosphatase alcaline préalablement modifiée.

La modification des molécules dont on veut qu'elles présentent des fonctions thiol libres peut être effectuée par réaction avec des réactifs amenant de telles fonctions tels que le réactif de Traut (Pierce) encore appelé imminothiolane.

Dans le cas de la phosphatase alcaline, la modification est effectuée en présence du réactif de TRAUT (Pierce).

La quantité de marqueur à utiliser dépend du nombre de fonctions maléimide que l'agent de couplage de l'invention utilisé contient. Elle pourra facilement être déterminée par l'homme du métier.

Bien entendu, les exemples donnés ci-dessus, d'une part pour les molécules présentant au moins une fonction aldéhyde et/ou cétone, et d'autre part pour les molécules présentant au moins une fonction thiol libre, ne sont pas limitatifs et sont interchangeables. En d'autres termes, par exemple, un agent de couplage peut être couplé, via sa fonction maléimide, à une molécule d'intérêt biologique modifiée pour inclure au moins une fonction thiol libre et, *via* sa fonction aminooxyalkylène, à un marqueur modifié pour inclure au moins une fonction aldéhyde et/ou cétone.

Les deux types de molécules ayant les fonctions telles que décrites ci-dessus liées aux agents de couplage de l'invention forment des conjugués qui constituent un autre objet de l'invention.

Selon un mode de réalisation de l'invention, les conjugués de l'invention remplissent au moins l'une des conditions suivantes :
- la molécule présentant au moins une fonction aldéhyde et/ou cétone est une molécule d'intérêt biologique, en particulier la glycoprotéine gp160 préalablement oxydée et
- la ou les molécules présentant au moins une fonction thiol libre sont des marqueurs, en particulier de la phosphatase alcaline préalablement modifiée.

Les conjugués ainsi obtenus peuvent être utilisés dans les méthodes de diagnostic *in vitro* de maladies impliquant une reconnaissance d'un couple ligand/anti-ligand, la molécule d'intérêt biologique constituant le ligand.

En effet, les conjugués de l'invention permettent de déterminer, par exemple dans un échantillon biologique, la présence d'un anti-ligand qui se fixera au ligand dudit conjugué, la révélation de la fixation étant réalisée grâce au marqueur dudit conjugué.

Les exemples de couples ligand/anti-ligand sont bien connus de l'homme du métier, ce qui est le cas par exemple des couples suivants : biotine/streptavidine, haptène/anticorps, antigène/anticorps, peptide/anticorps, sucre/lectine, polynucléotide/complémentaire du polynucléotide, étant entendu que l'un des éléments de ces couples constitue la molécule d'intérêt biologique.

Les méthodes diagnostic *in vitro* pouvant mettre en oeuvre les conjugués de l'invention sont notamment les méthodes de type sandwich et sont largement connues de l'homme du métier. A titre d'exemple, on peut citer la méthode ELISA, la méthode ELOSA, la méthode ELISPOT et la méthode Westem-blot.

Les maladies pouvant être diagnostiquées avec les complexes de l'invention ne sont pas limitées et comprennent toutes maladies révélées par la présence d'un marqueur spécifique de la maladie, du type molécule d'intérêt biologique, pour lequel il existe un partenaire de liaison. A titre d'exemple, on peut citer les maladies virales telles le SIDA, et les cancers solides telles que le cancer du sein ou de la prostate.

Les agents de couplage de l'invention, tels que définis précédemment, permettent d'améliorer le diagnostic *in vitro* de maladies qui met en oeuvre un couplage direct d'une molécule d'intérêt biologique et d'un ou plusieurs marqueurs, sans biotinylation intermédiaire de ladite molécule d'intérêt, du fait de la longueur de leur bras espaceurs et de la bonne stabilité des conjugués formés. De plus, l'utilisation des agents de couplage de l'invention permet d'amplifier la révélation de la liaison ligand/anti-ligand car lesdits agents de couplage peuvent se lier avec jusqu'à huit marqueurs tout en résolvant le problème de l'encombrement stérique de molécules en grand nombre.

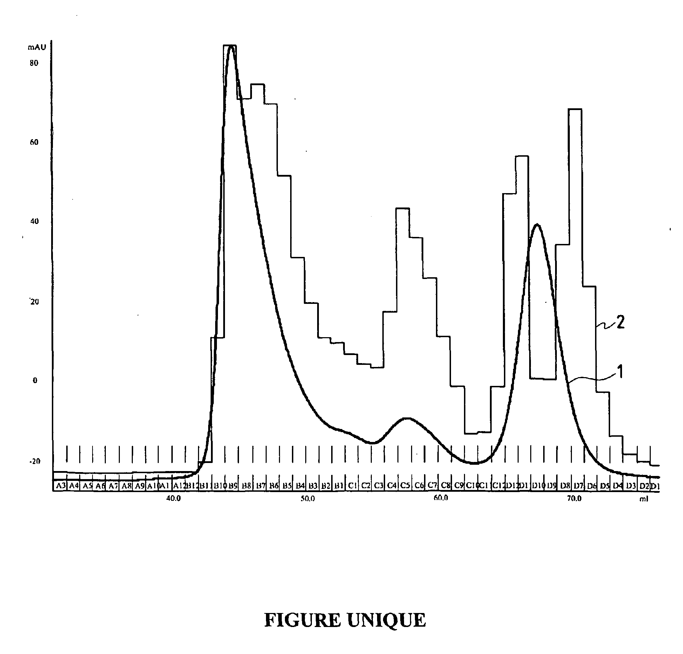

L'invention sera mieux comprise à l'aide de la figure 1 annexée, laquelle montre le profil enzymatique en utilisant un conjugué de l'invention, ainsi qu'à l'aide des exemples suivants qui sont donnés à titre illustratif et non limitatif.

### EXEMPLES

### Exemple 1 :

Composé de formule (I) dans laquelle m = 1, Y = -C(O)-NH₂, o=1, n=0, p=2, s=0 et B représente :

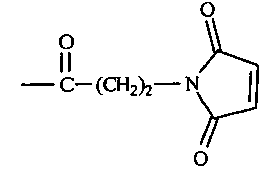

On a disposé dans un réacteur d'un synthétiseur automatique 431 A (Applied Biosystems) 282 µmoles de la résine Fmoc-RINK-Amide-MBHA (Novabiochem). On a préparé quatre cartouches des 3 synthons I, II et IV contenant 1/3 de mmole de chaque synthon comme suit :
- une cartouche contenant 166,4 mg (environ 333 µmol) du synthon suivant (correspondant au synthon I) : 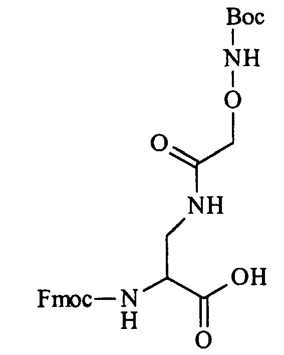 où Boc signifie t-butyloxycarbonyle et Fmoc signifie fluorénylméthoxycarbonyle,
- deux cartouches contenant chacune 128,5 mg (environ 333 µmol) du synthon II: 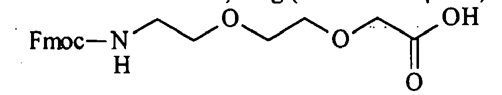 où Fmoc est tel que défini ci-dessus, et :
- une cartouche contenant 56,4 mg (environ 333 µmol) du synthon IV :

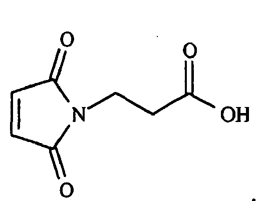

On a ensuite effectué quatre fois le cycle de couplage ci-dessous avec les cartouches ci-dessus, dans l'ordre :
- rinçage et solvatation de la résine par de la N-méthylpyrrolidinone (NMP),
- double traitement, pendant environ 4 min, puis pendant plus de 10 min, du synthon I (1^{er} passage) ou du complexe synthon(s)/résine (3^{ème} à 4^{ème} passage) par une solution de pipéridine à environ 40% dans la NMP pour déprotéger le groupement Fmoc,
- simultanément dilution dans un minimum de NMP et préactivation du synthon dans sa cartouche par addition d'un équivalent de l'agent de couplage hexafluorophosphatebenzotriazolyltetraméthyluronium (HBTU) sous la forme d'une solution de HBTU 0,2 M dans du *N*,*N*-diméthylformamide,
- vidange du réacteur et rinçages multiples de la résine avec de la NMP,
- transfert du contenu de la cartouche de synthon vers le réacteur et addition dans le réacteur d'environ 2,5 équivalents de la base tertiaire de couplage disopropyléthylamine (DIEA) sous la forme d'une solution de DIEA 1,67M dans de la NMP, et
- couplage pendant une heure et vidange du réacteur.

On a ensuite réalisé un cycle de rinçage de la résine en effectuant plusieurs rinçages par de la NMP, puis par du dichlorométhane (DCM).

On a ensuite effectué la déprotection et le décrochage de l'agent de couplage en le mettant à réagir à température ambiante pendant une heure dans environ 6,5 ml d'une solution d'acide trifluoroacétique (TFA) à 5% d'eau, puis en transférant la phase liquide dans un récipient contenant environ 100 ml d'éther éthylique à -10°C et en rinçant la résine par environ 4 ml de TFA et 4 ml de DCM, ces dernières phases liquides ayant été ajoutées à la première dans le récipient d'éther.

On a centrifugé le récipient contenant l'agent de couplage à 3500 tr/min à -5°C pendant 10 min et on a éliminé le surnageant éthéré. On a recommencé à deux reprises cette opération après addition d'éther.

Après séchage sous vide, on a obtenu 0,14 g de mélange brut.

On a vérifié la présence de l'agent de couplage dans le mélange brut en reprenant le mélange dans de l'eau déminéralisée MilliQ (Système Waters) et en analysant une quantité aliquote de ce mélange par HPLC BECKMANN (colonne de chromatographie de phase inverse C₁₈ VYDAC, éluant E_{A} solution d'eau déminéralisée MilliQ à 0,1% de TFA, éluant E_{B} solution de 95% d'acétonitrile et de 5% d'eau MilliQ à 0, 1 % de TFA, débit de purification 1 ml/min) en procédant à un gradient de 0 à 80% d'acétonitrile en 30 minutes. Dans de telles conditions, l'agent de couplage a un temps de rétention de l'ordre de 9,7 min (pic de volume mort vers 3 min).

On a purifié l'agent de couplage par HPLC semi-préparative BECKMAN (colonne de chromatographie de phase inverse C₁₈ VYDAC, éluant E_{A} solution d'eau déminéralisée MilliQ à 0,1% de TFA, éluant E_{B} solution de 95% d'acétonitrile et de 5% d'eau MilliQ à 0,1% de TFA , débit de purification 22 ml/min). Le gradient programmé pour cette purification a été le suivant :
- 0% d'éluant E_{B} de t = 0 à 10 min,
- passage à 5% de l'éluant E_{B} de t = 10 à 11 min et
- passage à 19% de l'éluant E_{B} de t = 11 à 41 min.

On a prélevé des fractions à partir de t=16,5 min à raison de 0,2 min par tube et de 40 tubes. On a analysé les tubes 11 à 40 en isocratique pendant 10 min à 11% d'éluant E_{B} sur le système analytique.

On a rassemblé les fractions les plus pures et on les a lyophilisées dans un lyophilisateur VIRTIS. Après lyophilisation et pesée, on a repris le produit dans l'eau MilliQ et aliquoté en tube Eppendorf.

### Exemple 2 :

Composé de formule (I) dans laquelle m = 1, Y = -C(O)-NH₂, o = 1, n = 0, p = 2, r = 4, s=1, q=0 et A et B sont identiques et représentent : 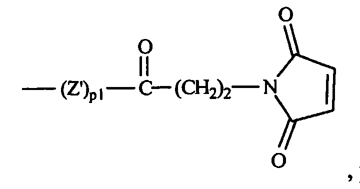 p1 étant égal à 2

Pour préparer le composé du titre, on a répété le mode opératoire décrit dans l'exemple 1, à ceci prêt qu'on est parti de 153 µmol du produit réactionnel entre la résine, une cartouche du synthon I et deux cartouches du synthon II tels que décrits dans l'exemple 1 et on a utilisé les cartouches de synthon suivantes :
- une cartouche contenant environ 166µmol du synthon suivant (correspondant au synthon III) : 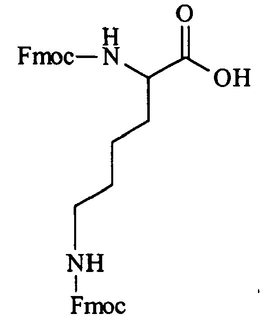 où Fmoc est tel que défini ci-dessus,
- quatre cartouches contenant chacune environ 333 µmol du synthon II: 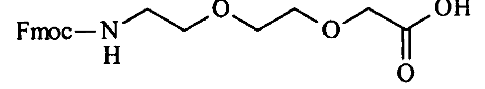 où Fmoc est tel que défini ci-dessus, et
- deux cartouches contenant environ 333 µmol du synthon IV suivant :

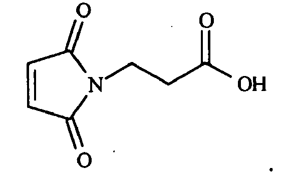

On a ainsi obtenu un produit sous forme de gomme qu'on a purifié comme indiqué ci-dessus, à ceci près qu'on a effectué le cycle de gradient suivant:
- 10% d'éluant E_{B} de t = 0 à 10 min,
- passage à 15% de l'éluant E_{B} de t = 10 à 11 min et
- passage à 27% de l'éluant E_{B} de t = 11 à 41 min..

### Exemple 3 : Réparation de l'intermédiaire activé gp 160/agent de couplage de l'invention

On a dialysé la glycoprotéine gp160 (ABL) une nuit à 2-8°C contre un tampon acétate 50mM à pH 4,5 en présence de SDS 0,01%. On a oxydé la glycoprotéine ainsi obtenue avec du NaIO₄ 5 mM à l'obscurité pendant 15 min à 18-25°C. On a bloqué la réaction par l'éthylèneglycol (1/200) et on a dialysé la protéine oxydée contre une solution de tétraborate de sodium 25 mM à pH 5,5.

On a préparé une solution de l'agent de couplage tel que préparé dans l'exemple 2 à 2 mg/ml dans une solution de tétraborate de sodium.

On a mélangé la solution de l'agent de couplage à la glycoprotéine oxydée à raison de 20 mol d'agent de couplage par unité de 160 000 Da de glycoprotéine et on a fait incuber une heure à 18-25°C sous agitation pour réaliser le couplage.

On a réalisé une dialyse du mélange obtenu contre un tampon phosphate 50 mM, NaCl 150 mM, à PH 6,8.

### Exemple 4 : Préparation du conjugué gp 160/agent de couplage/phosphatase alcaline

La phosphatase alcaline a été modifiée pour inclure des groupements thiol libres de la façon suivante : on a effectué une dialyse de la phosphatase alcaline (Biozyme) contre un tampon phosphate 10 mM, EDTA 5 mM, à pH 8. On a préparé une solution de réactif de Traut (Pierce) à 3 mg/ml dans un tampon phosphate 10 mM, EDTA 5 mM, à pH 8 et on a laissé incuber 2 heures à 18-25°C. On a dessalé le mélange réactionnel sur une colonne de Sephadex G-25 (Pharmacia).

On a mélangé 1 mg d'intermédiaire activé obtenu à l'exemple 3 avec 3 mg de la phosphatase alcaline modifiée et on a fait incuber une nuit à 2-8°C sous agitation. On a bloqué la réaction (groupements maléimide) par addition d'une solution de β-mercaptoéthanol 10mM dans le tampon à pH 6,8 et on a laissé incubé 15 min à température ambiante. On a bloqué la réaction (fonctions thiol résiduelles) avec une solution de N-éthylmaléimide (Sigma) dans le tampon à pH 6,8 et on a laissé incubé 15 min à température ambiante. On a ensuite dialysé 2 fois 45 min à température ambiante et une nuit à 2-8°C contre le tampon PBS.

On a alors purifié le conjugué à l'aide de la colonne de chromatographie Superdex 200 (Pharmacia) (éluant : PBS, azide avec SDS 0,01%, débit : 1 ml/min, fractions : 1 ml).

On a recherché l'activité enzymatique des fractions de chromatographie à l'aide d'un test sur microplaques consistant à prélever 25 µl de chaque fraction, à compléter avec du PBS jusqu'à obtenir 100 µl, à ajouter 100 µl de pNPP, à laisser incuber pendant 5 min, à bloquer la réaction par 20 µl de NaOH 0,5 N et à mesurer la densité optique.

Le profil d'activité enzymatique de ce test est donné sur la Figure unique qui met en évidence le bon niveau de marquage obtenu. Les différentes fractions sont mentionnées au-dessus de l'axe des abscisses.

Les densités optiques du conjugué seul à 280 nm (courbe 1), d'une part, et du conjugué après réaction enzymatique à 405 nm (courbe 2), d'autre part, sont mesurées.

### Exemple 5 : Comparaison de la sensibilité de détection d'un conjugué de l'invention et d'un conjugué obtenu avec un agent de couplage de Pierce

On a utilisé un conjugué obtenu avec l'agent de couplage de l'exemple 1 et un conjugué obtenu avec l'agent de couplage de Pierce (EMCH, tel que décrit ci-après) et on a comparé la sensibilité en terme de détection obtenue avec ces différents conjugués.

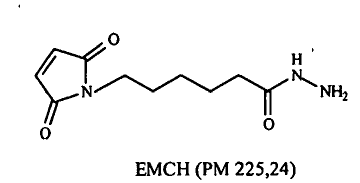

Pour ce faire, on a introduit dans une cuve réactionnelle 150 µl de particules magnétiques de 1 µm, en suspension à 1% et diluées au 1/50 dans du tampon (Tris 200mM - NaCl 150 mM pH 8,5 - BSA 10 g/l - Tween 20 5%, NaN₃ 0,9 g/l), fonctionnalisées par des groupements -COOH et sensibilisées avec la glycoprotéine gp160 du virus VIH-1, ainsi que 150 µl d'échantillon. Les échantillons testés sont soit un pool de sérums négatifs VIH-1 (VIH-1 -), soit des échantillons contenant le virus VIH-1 (VIH-1 +) dilué dans un pool de sérums négatifs. On a laissé incubé 15 min à 37°C.

On a ajouté ensuite 150 µl d'un réactif contenant les conjugués gp160/agent de couplage/phosphatase alcaline préparés selon le mode opératoire décrit dans l'exemple 4 en utilisant l'agent de couplage approprié et dilués pour obtenir une concentration d'environ 2 µg/ml dans un tampon Tris 100 mM - NaCl 300 mM pH 7,4 - Mannitol 2,5 g/l- NaN₃ 0,9 g/l- lait Régilait® 2,5 g/l- BSA 2,5 g/l- ZnCl₂ 0,1 mM- MgCl₂ 1 mM. On a laissé incuber 15 min à 37°C.

On a lavé à trois reprises avec la solution de lavage constituée par un tampon citrate pH 6 contenant du NaCl, un détergeant, un antimousse et un antimicrobien (MAGIA, Biotrol).

On a ajouté dans la cuve réactionnelle le substrat de luminescence Lumiphos 530 (Lumigen) et on a laissé incubé 15 min à 37°C.

On a lu la luminescence en unité RLU (Relative Luminescence Unit) à l'aide du photomultiplicateur H7155 (HAMAMATSU) et on a calculé le rapport de luminescence entre les échantillons contenant le virus et ceux sans le virus.

Les résultats sont donnés dans le tableau 1 ci-dessous.

**Tableau 1 : test de sensibilité**

| Conjugué | Luminescence RLU | | | | |
|---|---|---|---|---|---|
| | VIH-1 - | VIH-1 + (1/2000) | VIH-1 + (1/300) | Rapport | Rapport |
| | a1 | a2 | a3 | a2/a1 | a3/a1 |
| avec exemple 1 | 1170 | 58342 | 3409 | 49,9 | 2,9 |
| avec EMCH | 8103 | 134104 | 10621 | 16,6 | 1,3 |
| avec EMCH (dilution d'un facteur 2) | 3731 | 61526 | 5033 | 16,5 | 1,35 |

Les rapports obtenus avec le conjugué de l'invention et avec un conjugué préparé avec EMCH tels qu'indiqués ci-dessus montrent clairement l'amélioration de la sensibilité de détection lorsqu'on utilise un agent de couplage de l'invention, que la dilution soit importante ou non (2 dernières colonnes du tableau).

Il est à noter que, pour obtenir un niveau de détection contrôle (bruit de fond) comparable à celui obtenu avec le conjugué de l'invention, on a dilué d'un facteur 2 le conjugué préparé à partir de EMCH (dernière ligne). On a ainsi vérifié que, à bruit de fond comparable, le rapport de luminescence reste faible avec le conjugué préparé à partir de l'agent de couplage de l'art antérieur.

### Exemple 6 : Comparaison de la stabilité d'un conjugué de l'invention et d'un conjugué obtenu avec l'agent de couplage EMCH

Pour étudier la stabilité des conjugués, on a laissé vieillir les conjugués dilués comme indiqué dans l'exemple 5, dans une étuve à 37°C pendant 8, 15 et 30 jours et on a comparé leur comportement vis-à-vis des mêmes préparations, mais conservées à 4°C. Les résultats, en terme de pourcentage, sont indiqués dans le tableau 2 ci-dessous.

**Tableau 2 : test de stabilité**

| | 8 jours à + 37°C | 15 jours à + 37°C | 30 jours à + 37°C |
|---|---|---|---|
| Conjugué de l'invention | - 8,7 % | - 13,2 % | -37,1 % |
| Conjugué avec EMCH | -36,7% | - 57,3 % | -56,8% |

Les résultats du tableau 2 montrent clairement qu'en condition forcée de vieillissement, le conjugué préparé avec un agent de couplage de l'invention est bien plus stable que le conjugué préparé avec un agent de couplage de l'art antérieur.

### Exemple 7 : Préparation de l'agent de couplage décrit par Mikolajczyk S. et al. (supra)

On a réalisé l'ensemble des opérations de préparation sur un synthétiseur automatique de peptides 431 A (APPLIED BIOSYSTEMS) à température ambiante, selon le schéma suivant.

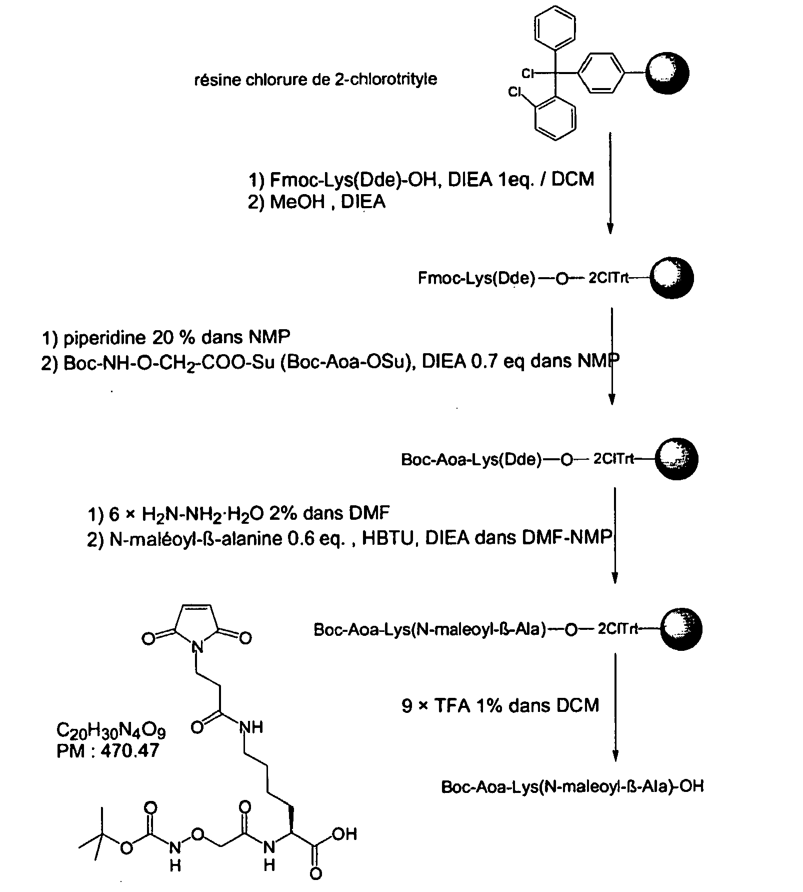

Dans le réacteur, on a disposé d'environ 0,68 mmole (en équivalent chlorure) de résine chlorure de 2-chlorotrityle (Novabiotech) très acidolabile.

Selon un protocole semi-automatique, on a fonctionnalisé cette résine par addition d'une solution contenant 1 mmole (environ 535 mg) de Fmoc-Lys(Dde)-OH (Dde : (4,4-diméthyl-2,6-dioxocyclohex-1-ylidène)éthyle, NOVABIOCHEM) et 1 mmole de diisopropyléthylamine (DIEA, ALDRICH) dans 6 ml de dichlorométhane (DCM, ABI) pour fixer la lysine protégée sur la résine par un lien chimique de type ester.

Après 35 minutes sous vortex, on a injecté dans le réacteur 0,5 ml de méthanol anhydre (MERCK) et 5 ml d'une solution 5M de 2,5 mmoles de DIEA dans de la N-methyl-pyrrolidone (NMP, ABI).

Après plusieurs lavages de la résine au DCM, on a fait agir sous agitation une solution à 20-30% de pipéridine (ALDRICH) dans la NMP, 2 fois 5 minutes. On a ensuite rincé la résine plusieurs fois à la NMP et au DCM.

On a introduit sur la résine une solution de 6 ml de DCM et d'environ 0,69 mmole de Boc-Aoa-Osu et 0,25 ml de la solution 5M de 1,25 mmole de DIEA dans la NMP déjà citée plus haut. La réaction de couplage a duré 55 minutes sous agitation. On a ensuite rincé plusieurs fois la résine à la NMP.

Pour effectuer la déprotection sélective du groupement Dde, on a traité la résine 6 fois 3 minutes par 8 ml d'une solution d'hydrate d'hydrazine (ALDRICH) à 2% dans le diméthylformamide (DMF ALDRICH). On a ensuite procédé à plusieurs rinçages à la NMP.

Pour coupler la N-maléoyl-ß-alanine, on a mis à réagir 0,6 mmole de N-maléoyl-ß-alanine (FLUKA), soit environ 100 mg repris dans environ 5 ml de NMP, sur la résine avec 1 ml d'une solution 0,6M de 1 équivalent de l'agent de couplage HBTU (QUANTUM APPLIGENE) dans le DMF et 300 µl de solution de DIEA 5M de 2,5 équivalents de DIEA dans la NMP. Le couplage sous agitation a duré environ 40 minutes, après quoi la résine a été rincée plusieurs fois à la NMP, puis au DCM.

On a décroché l'agent de couplage de la résine par une acidolyse douce sur la résine traitée à température ambiante 9 fois 2 minutes par 5,5 ml d'une solution à 1% d'acide trifluoroacétique (TFA, ACROS) dans du DCM. On a collecté chacun de ces volumes et on les a ajoutés à 80 ml d'éther diéthylique (SDS) préalablement refroidi à -10°C. On a fait évaporer le mélange éthéré à l'évaporateur rotatif (type BÜCHI) par fractions de quelques dizaines de ml ajoutées à un volume de 50 ml de cyclohexane (PROLABO) pour éliminer l'éther, le DCM, le TFA et le cyclohexane. On a récupéré 0,14 g de produit, ce qui représente un rendement brut de 44%.

On a purifié et analysé l'agent de couplage de la façon suivante:
On a lentement repris tout le produit ainsi obtenu dans 2 ml d'acétonitrile (ACN, MERCK). Lorsque la solubilisation a été complète, on a ajouté 1 ml d'eau déminéralisée et on a transféré dans un flacon à échantillons. On a rincé le ballon avec un mélange de 2 ml d'éthanol et 1 ml d'eau, qui ont été ajoutés au premier volume de reprise (3 ml). On a recommencé cette opération avec de l'eau pour obtenir finalement l'agent de couplage dans environ 50 ml d'un mélange limpide à 4% d'ACN et 4 % d'éthanol. On a injecté ce volume en plusieurs fois dans un appareil de chromatographie liquide haute performance HPLC semi-préparative BECKMAN (munie de pompes modèle 126 et détecteur UV modèle 166), avec une colonne phase inverse C₁₈ VYDAC (référence 218TP152022) à un débit de 22 ml.min⁻¹. La longueur d'onde de travail est de 214 nm. Les 2 éluants utilisés pour cette purification sont A) l'eau déminéralisée «milliQ» (système WATERS) à 0,1% de TFA et B) un mélange 95% ACN - 5% eau à 0,1% de TFA.

On a maintenu la colonne à 10% de B) pendant 10 minutes pour permettre l'injection de la solution à purifier, puis on est passé à 20% de B) en 1 minute et on a poursuivi par un gradient plat de 20 à 31 % de B) en 30 minutes. On a prélevé en sortie de colonne 12 fractions à partir de 19,3 minutes à raison de 0,25 minute par tube.

On a analysé une aliquote de chaque fraction par HPLC analytique BECKMAN avec les mêmes éluants que précédemment, mais sur une colonne plus petite phase inverse C₁₈ VYDAC (référence 218TP54), avec un débit de 1 ml.min⁻¹ et une proportion d'éluant B) constante (isocratique) de 26% pendant 10 minutes. On a rassemblé les fractions jugées les plus pures et on les a lyophilisées pour obtenir 12,6 mg de produit, soit un rendement apparent avant dosage final de l'agent de couplage de 2,7% depuis le début de la synthèse. On a repris l'agent de couplage en solution aqueuse à 50% d'ACN et on a aliquoté en fractions de 580 µg chacune (500 µg théoriques dont on a vérifié que ces fractions étaient de 580 µg), lesquelles ont été lyophilisées. On a confirmé la structure de l'agent de couplage protégé par spectrométrie de masse PE-SCIEX (modèle API100) à M=470,4 daltons (théorique moyen 470,48 et mono-isotopique 470,20).

On a déprotégé l'agent de couplage par mise en réaction d'une des aliquotes de 580 µg avec 100 µl de TFA sous agitation pendant 30 minutes à température ambiante. On a ensuite transféré cette solution dans un récipient en verre de 2 ml adapté à la centrifugeuse auquel ont été ajoutés 3 rinçages de 100 µl de DCM. On a procédé à une évaporation dans une centrifugeuse sous vide SPEED VAC^{®} en 15 minutes. On a alors obtenu 580 µg de l'agent de couplage avec un rendement de à 4,6%.

### Exemple 8 : Comparaison de la sensibilité de détection d'un conjugué de l'invention et d'un conjugué obtenu avec l'agent de couplage obtenu à l'exemple 7

On a préparé des conjugués gp 160/agent de couplage/phosphatase alcaline selon le mode opératoire indiqué dans les exemples 3 et 4, avec l'agent de couplage de l'invention décrit à l'exemple 1 et avec l'agent de couplage de Mikolajczyk S. et al. tel que décrit à l'exemple 7, à ceci près que, pour l'agent de couplage de l'art antérieur :
i) on l'a déprotégé comme suit, par rapport à l'exemple 7 : on a repris une aliquote lyophilisée de l'agent de couplage (580 µg) et on l'a mise à réagir avec 100 µl d'acide trifluoroacétique pendant 30 minutes à température ambiante et sous agitation. Ensuite, on a transféré ce mélange réactionnel dans un flacon compatible avec la centrifugeuse sous vide « SPEED VAC » et on a ajouté les produits de 2 rinçages de dichlorométhane (2×100 µl). On a procédé à un séchage de 20 minutes sur cet appareil (sans chauffer l'enceinte) pour obtenir un culot légèrement jaune dudit agent de couplage,
ii) dans l'étape d'oxydation de la gp160 (exemple 3), on a utilisé une solution de NaIO₄ 0,2M et on a bloqué avec une solution d'éthylèneglycol au 1/150.
Pour déterminer la sensibilité de détection, on a repris le mode opératoire décrit dans l'exemple 5, à ceci près qu'on a utilisé 250 µl de particules magnétiques et une concentration de la solution de conjugué égale à 0,5 gg.ml⁻¹, ainsi que des sérums faiblement positifs en VIH-1 (VIH-1 + faible), moyennement positifs en VIH-1 (VIH-1 + moyen) et fortement positifs en VIH-1 (VIH-1 + fort).

Par sérums faiblement positifs, on entend des sérums faiblement riches en anticorps anti-VIH, c'est-à-dire dont la mesure de détection est proche du seuil lorsqu'on l'analyse avec un kit commercial. On entend par sérums moyennement positifs des sérums moyennement riches en anticorps et on entend par sérums fortement positifs des sérums fortement riches en anticorps.

La luminescence en unité RLU et le rapport de luminescence obtenus avec les deux composés sont donnés dans le tableau 3 ci-dessous.

**Tableau 3**

| Conjugué | Luminescence RLU | | | | | | |
|---|---|---|---|---|---|---|---|
| | VIH-1- | VIH-1 + faible | VIH-1 + moyen | VIH-1 + fort | Rapport | Rapport | Rapport |
| | a1 | a2 | a3 | a4 | a2/a1 | a3/a1 | a4/a1 |
| avec exemple 1 | 1291 | 18258 | 99444 | 655177 | 14,14 | 77,01 | 507,36 |
| avec exemple 7 | 2386 | 15688 | 90593 | 517137 | 6,57 | 37,97 | 216,74 |

Là-encore, les rapports obtenus avec le conjugué de l'invention et avec un conjugué préparé avec l'agent de couplage de Mikolajczyk S. et al. tels qu'indiqués ci-dessus montrent clairement l'amélioration de la sensibilité de détection lorsqu'on utilise l'agent de couplage de l'invention.

## Revendications

1. Agent de couplage de formule générale suivante : 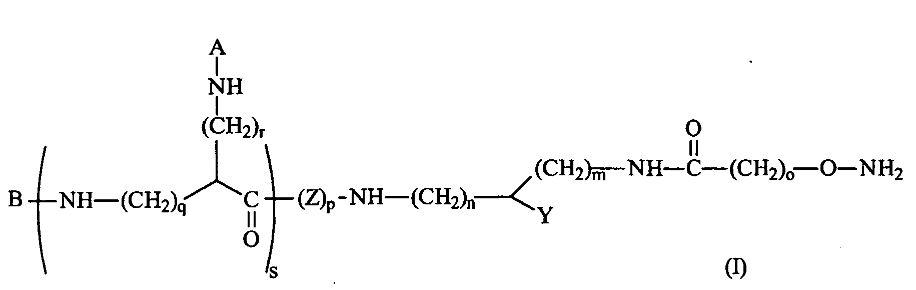 dans laquelle :
- Z représente -NH-(CH₂)₂-O-(CH₂)₂-O-CH₂-C(O)-
- A représente un groupe choisi parmi : 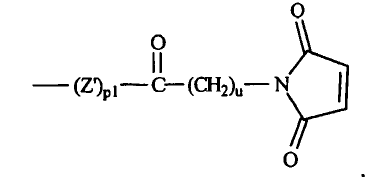 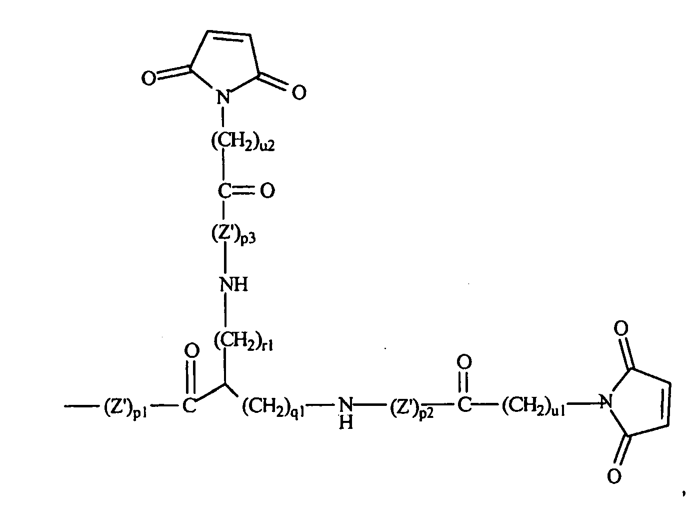 et 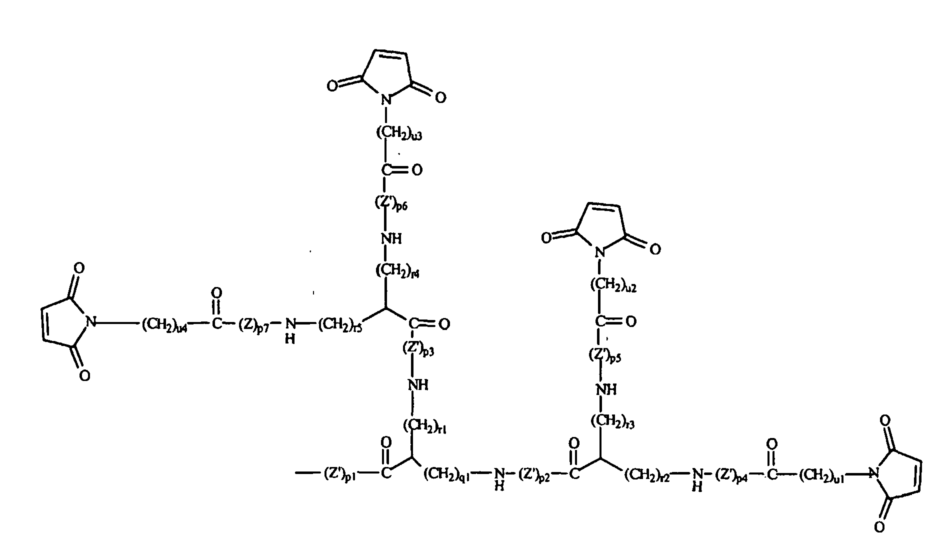 B représente un groupe choisi parmi : 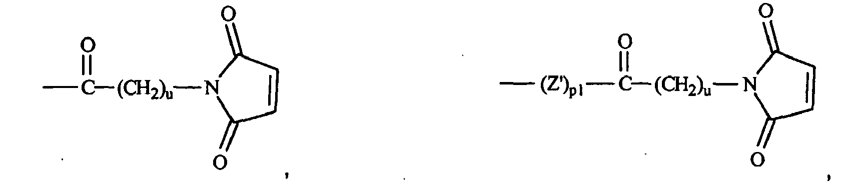 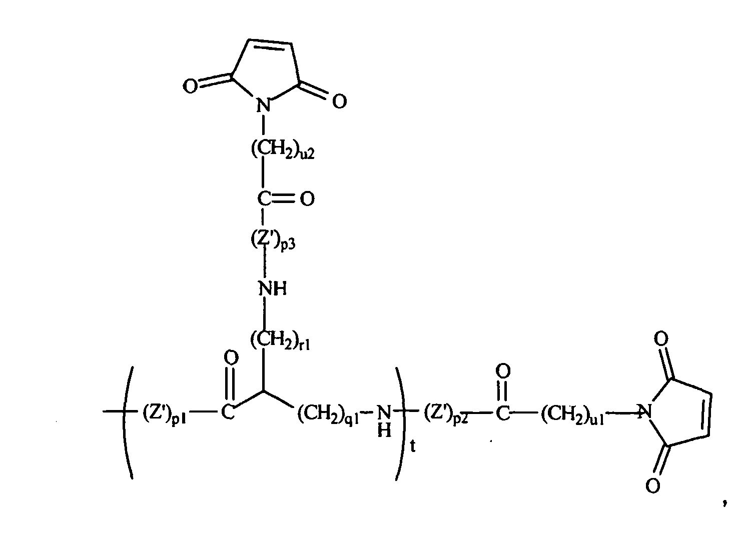 et 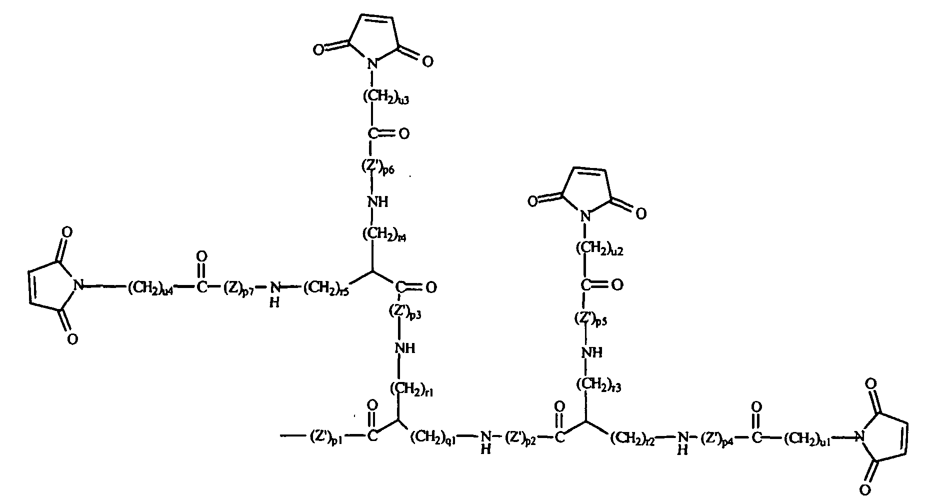
- Y représente un groupement qui se termine par -C(O)-NH₂ et qui est inerte vis-à-vis de la fonction aminooxyalkylène, du ou des groupes maléimide de B et éventuellement de A et du motif -(CH₂)₂-O-(CH₂)₂-O-CH₂- de Z,
- Z' représente -C(O)-CH₂-O-(CH₂)₂-O-(CH₂)₂-NH-,
- n est un nombre entier compris entre 0 et 3,
- m est un nombre entier compris entre 1 et 3,
- o est un nombre entier compris entre 1 et 3,
- p et p1 à p7 sont chacun indépendamment un nombre entier compris entre 1 et 4,
- q et q1 sont chacun indépendamment un nombre entier compris entre 0 et 3,
- r et r1 à r5 sont chacun indépendamment un nombre entier compris entre 2 et 5,
- s est un nombre entier égal à 0 ou 1,
- t est un nombre entier compris entre 1 et 7, et
- u et u1 à u4 sont chacun indépendamment un nombre entier compris entre 2 et 10.

2. Agent de couplage selon la revendication 1, **caractérisée en ce que** Y est -C(O)-NH₂.

3. Agent de couplage selon l'une des revendications 1 ou 2, **caractérisé en ce que** s est égal à 0.

4. Agent de couplage selon la revendication 3, **caractérisé en ce que** B représente : 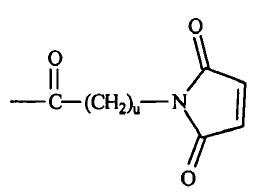 u étant tel que défini dans la revendication 1.

5. Agent de couplage selon l'une des revendications 1 ou 2, **caractérisé en ce que** A et B représentent : 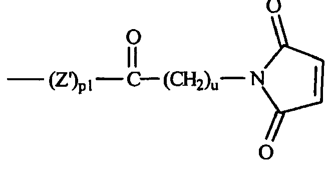 Z', p1 et u étant tels que définis dans la revendication 1.

6. Agent de couplage selon l'une des revendications 1 ou 2, **caractérisé en ce que** A et B représentent : 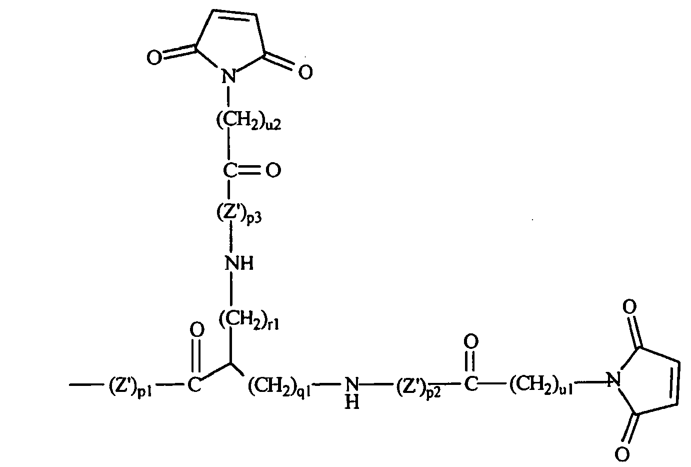 Z', p1, p2, p3, q1, r1, u1 et u2 étant tels que définis dans la revendication 1.

7. Agent de couplage selon l'une des revendications 1 ou 2, **caractérisé en ce que** A et B représentent : 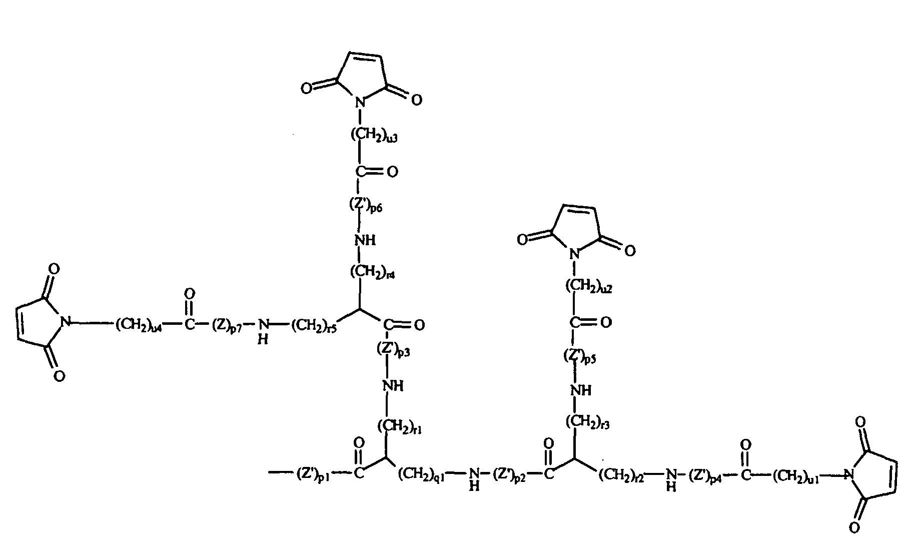 Z, Z', p1 à p7, q1, r1 à r5 et u1 à u4 étant tels que définis dans la revendication 1.

8. Agent de couplage selon l'une des revendications 1 ou 2, **caractérisé en ce que** A représente : 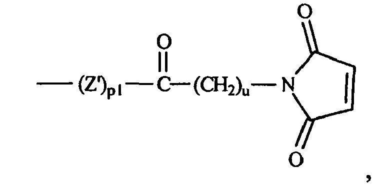 et
B représente : 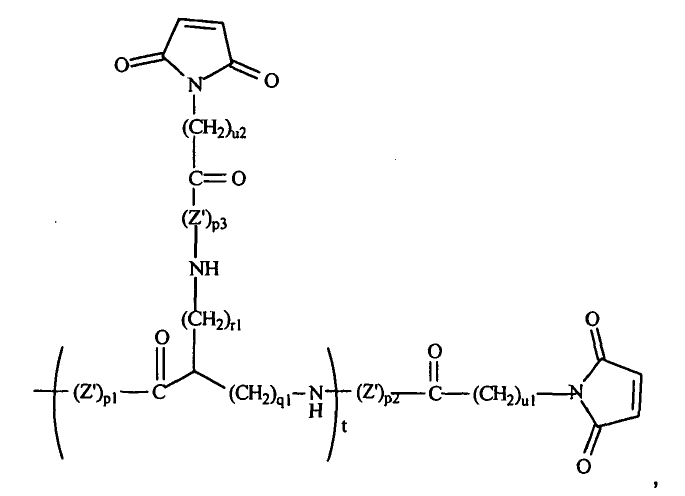 Z', t, p1 à p3, q1, r1, u, u1 et u2 étant tels que définis dans la revendication 1.

9. Agent de couplage selon l'une quelconque des revendications précédentes, **caractérisé en ce que** n est égal à 0.

10. Agent de couplage selon l'une quelconque des revendications précédentes, **caractérisé en ce que** m est égal à 1.

11. Agent de couplage selon l'une quelconque des revendications précédentes, **caractérisé en ce que** p et p1 à p7, le cas échéant, sont égaux à 2.

12. Agent de couplage selon l'une quelconque des revendications précédentes, **caractérisé en ce que** q et q1, le cas échéant, sont égaux à 0.

13. Agent de couplage selon l'une quelconque des revendications précédentes, **caractérisé en ce que** r et r1 à r5, le cas échéant, sont égaux à 4.

14. Agent de couplage selon l'une quelconque des revendications précédentes, **caractérisé en ce que** u et u1 à u4, le cas échéant, sont égaux à 2.

15. Agent de couplage selon l'une quelconque des revendications précédentes, **caractérisé en ce que** o est égal à 1.

16. Agent de couplage selon la revendication 1, **caractérisé en ce qu'**il est :
- le composé de formule (I) dans laquelle m = 1, Y = -C(O)-NH₂, o=1, n=0, p=2, s= 0 et B représente : 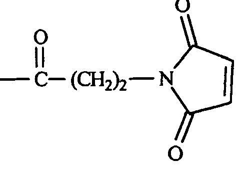 ou
- le composé de formule (I) dans laquelle m = 1, Y = -C(O)-NH₂, o = 1, n = 0, p = 2, r=4,s=1, q=0 et A et B sont identiques et représentent : 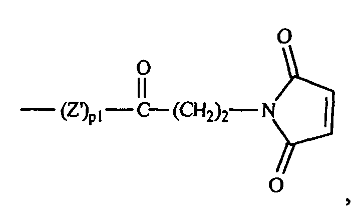
p1 étant égal à 2.

17. Intermédiaire activé constitué d'un agent de couplage selon l'une quelconque des revendications 1 à 16 et d'une molécule présentant à sa surface au moins une fonction aldéhyde et/ou cétone avant conjugaison.

18. Intermédiaire activé selon la revendication 17, **caractérisé en ce que** la molécule présentant à sa surface au moins une fonction aldhéhyde est une glycoprotéine oxydée au périodate.

19. Intermédiaire activé selon la revendication 18, **caractérisé en ce que** ladite glycoprotéine est la glycoprotéine gp160.

20. Intermédiaire activé constitué d'un agent de couplage selon l'une quelconque des revendications 1 à 16 et de une à huit molécules présentant en surface au moins une fonction thiol libre avant conjugaison.

21. Intermédiaire activé selon la revendication 20, **caractérisé en ce qu'**il contient de une à quatre molécules présentant en surface au moins une fonction thiol libre avant conjugaison.

22. Intermédiaire activé selon l'une des revendications 20 ou 21, **caractérisé en ce que** ladite ou lesdites autre(s) molécule(s) présentant en surface au moins une fonction thiol libre est (sont) un marqueur.

23. Intermédiaire activé selon la revendication 22, **caractérisé en ce que** le marqueur est la phosphatase alcaline préalablement modifiée pour inclure des fonctions thiol libres.

24. Conjugué constitué d'un agent de couplage selon l'une quelconque des revendications 1 à 16 lié à une molécule présentant à sa surface au moins une fonction aldéhyde et/ou cétone avant conjugaison et à une à huit molécules présentant en surface au moins une fonction thiol libre avant conjugaison .

25. Conjugué selon la revendication 24, **caractérisé en ce que** la molécule présentant à sa surface au moins une fonction aldéhyde et/ou cétone avant conjugaison est une molécule d'intérêt biologique.

26. Conjugué selon la revendication 25, **caractérisée en ce que** la molécule d'intérêt est la glycoprotéine gp160 préalablement oxydée.

27. Conjugué selon l'une quelconque des revendications 24 à 26, **caractérisé en ce que** la ou les molécules présentant en surface au moins une fonction thiol libre avant conjugaison sont des marqueurs.

28. Conjugué selon la revendication 27, **caractérisé en ce que** les marqueurs sont la phosphatase alcaline préalablement modifiée pour inclure des fonctions thiol libres.

29. Utilisation d'un conjugué selon l'une quelconque des revendications 24 à 28 dans les méthodes de diagnostic *in vitro* de maladies impliquant une reconnaissance d'un couple ligand-anti-ligand.

## Claims

1. Coupling agent having the following general formula: 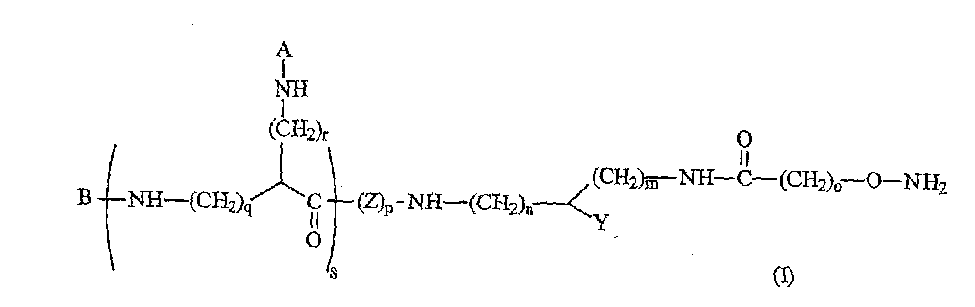 in which:
- Z represents -NH-(CH₂)₂-O-(CH₂)₂-O-CH₂-C(O)-
- A represents a group chosen from among; 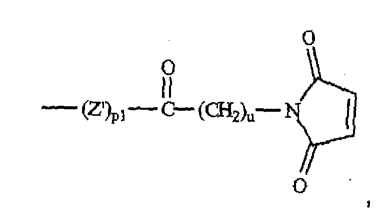 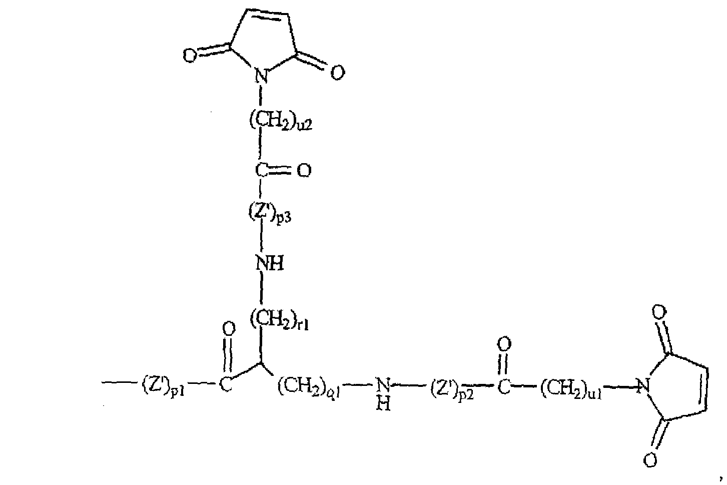 and 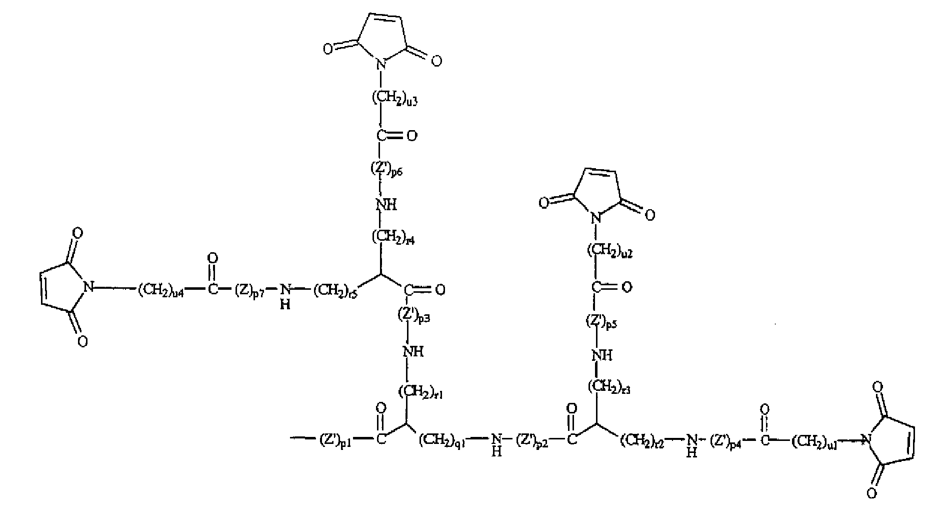 B represents a group chosen from among: 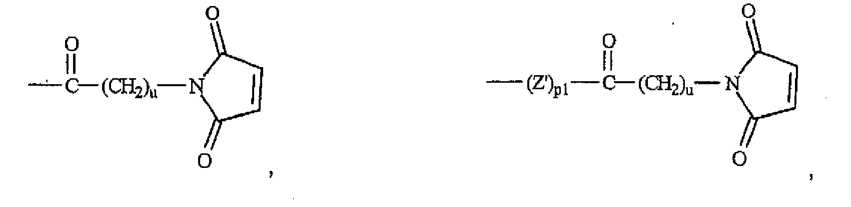 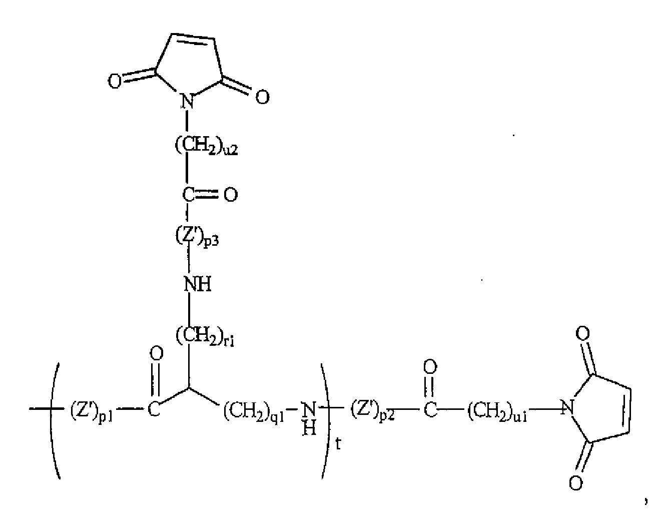 and 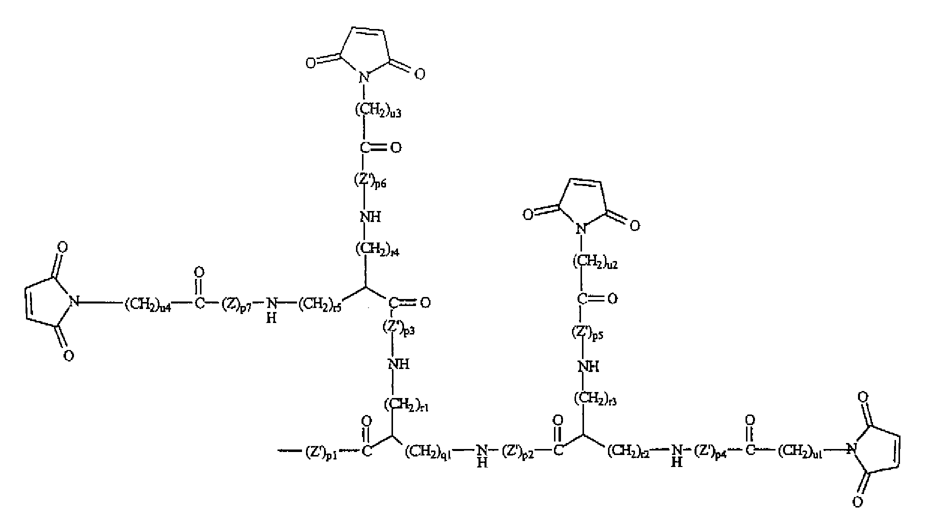
- Y represents a group ending in -C(O)-NH₂ and which is inert vis-à-vis the aminooxyalkylene function, the maleimide group or groups of B and optionally of A and vis-à-vis the pattern -(CH₂)₂-O-(CH₂)₂-O-CH₂- of Z,
- Z' represents -C(O)-CH₂-O-(CH₂)₂-O-(CH₂)₂-NH-,
- n is an integer lying between 0 and 3,
- m is an integer lying between 1 and 3,
- o is an integer lying between 1 and 3,
- p and p1 to p7 are each independently an integer lying between 1 and 4,
- q and q1 are each independently an integer lying between 0 and 3,
- r and r1 to r5 are each independently an integer lying between 2 and 5,
- s is an integer of 0 or 1,
- t is an integer lying between 1 and 7, and
- u and u1 to u4 are each independently an integer lying between 2 and 10.

2. Coupling agent as in claim 1, **characterized in that** Y is -C(O)-NH₂.

3. Coupling agent as in either of claims 1 or 2, **characterized in that** s equals 0.

4. Coupling agent as in claim 3, **characterized in that** B represents: 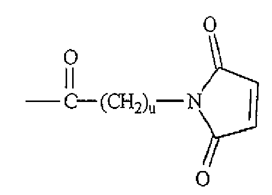 u being such as defined in claim 1.

5. Coupling agent as in either of claims 1 or 2, **characterized in that** A and B represent: 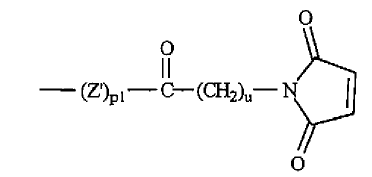 Z', p1 and u being such as defined in claim 1.

6. Coupling agent as in either of claims 1 or 2, **characterized in that** A and B represent: 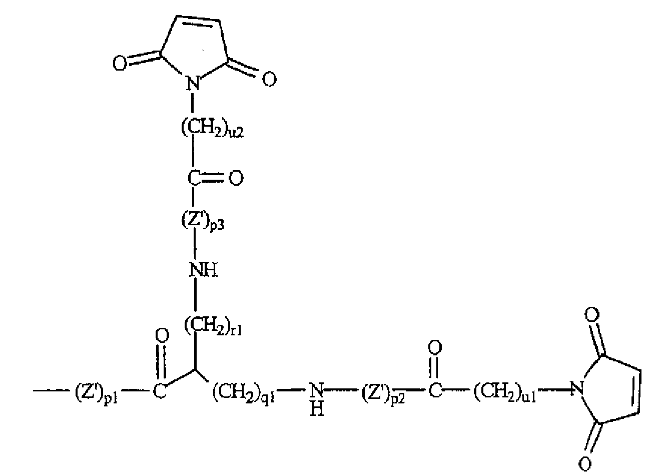 Z', p1, p2, p3, q1, r1, u1 and u2 being such as defined in claim1.

7. Coupling agent as in either of claims 1 or 2, **characterized in that** A and B represent: 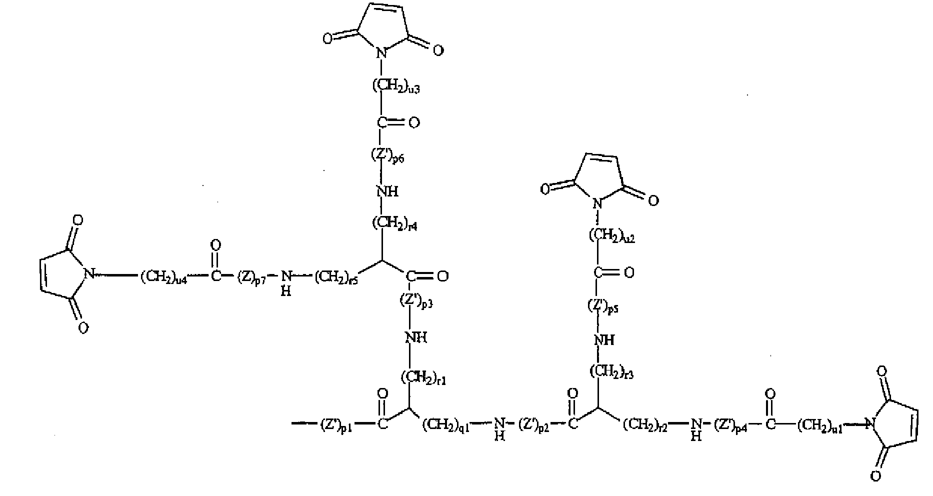 Z, Z', p1 to p7, q1, r1 to r5 and u1 to u4 being such as defined in claim 1.

8. Coupling agent as in either of claims 1 or 2, **characterized in that** A represents: 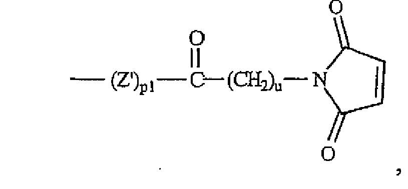 , and B represents: 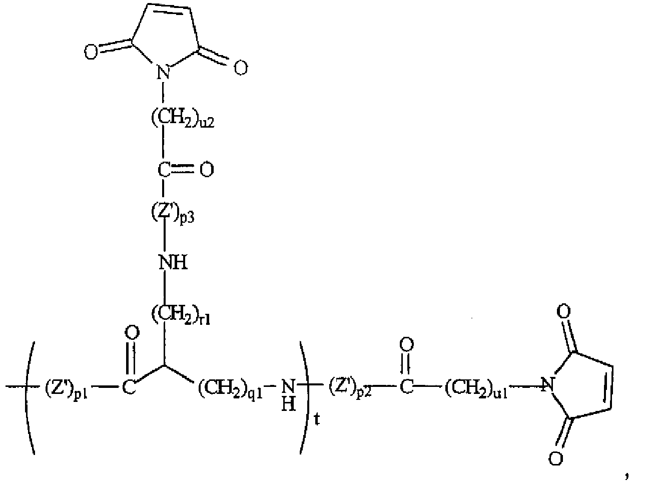 Z', t, p1 to p3, q1, r1, u, u1 and u2 being such as defined in claim1.

9. Coupling agent as in any of the preceding claims, **characterized in that** n equals 0.

10. Coupling agent as in any of the preceding claims, **characterized in that** m equals 1.

11. Coupling agent as in any of the preceding claims, **characterized in that** p and p1 to p7, when applicable, equal 2.

12. Coupling agent as in any of the preceding claims, **characterized in that** q and q1, when applicable, equal 0.

13. Coupling agent as in any of the preceding claims, **characterized in that** r and r1 to r5, when applicable, equal 4.

14. Coupling agent as in any of the preceding claims, **characterized in that** u and u1 to u4, when applicable, equal 2.

15. Coupling agent as in any of the preceding claims, **characterized in that** o equals 1.

16. Coupling agent as in claim 1, **characterized in that** it is:
- the compound of formula (I) in which m = 1, Y = -C(O)-NH₂, o=1, n=0, p=2, s=0 and B represents: 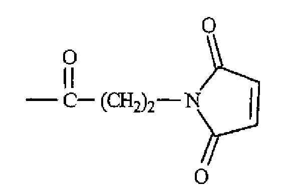 or
- the compound of formula (I) in which m = 1, Y = -C(O)-NH₂, o=1, n=0, p=2, r=4, s=1, q=0 and A and B are identical and represent: 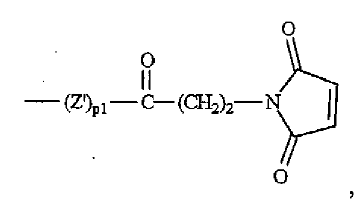 p1 equalling 2.

17. Activated intermediate formed of a coupling agent as in any of claims 1 to 16 and of a molecule having on its surface at least one aldehyde and/or ketone function before conjugation.

18. Activated intermediate as in claim 17, **characterized in that** the molecule having on its surface at least one aldehyde function is a glycoprotein oxidized with periodate.

19. Activated intermediate as in claim 18, **characterized in that** said glycoprotein is the gp160 glycoprotein.

20. Activated intermediate consisting of a coupling agent as in any of claims 1 to 16 and of one to eight molecules having on their surface at least one free thiol function before conjugation.

21. Activated intermediate as in claim 20, **characterized in that** it contains from one to four molecules having on their surface at least one free thiol function before conjugation.

22. Activated intermediate as in either of claims 20 or 21, **characterized in that** said other molecule(s) having on their surface at least one free thiol function is(are) a marker.

23. Activated intermediate as in claim 22, **characterized in that** the marker is alkaline phosphatase previously modified to include free thiol functions.

24. Conjugate formed of a coupling agent as in any of claims 1 to 16 linked to a molecule having on its surface at least one aldehyde and/or ketone function before conjugation, and to one to eight molecules having on their surface at least one free thiol function before conjugation.

25. Conjugate as in claim 24, **characterized in that** the molecule having on its surface at least one aldehyde and/or ketone function before conjugation is a molecule of biological interest.

26. Conjugate as in claim 25, **characterized in that** the molecule of interest is the gp160 glycoprotein previously oxidized.

27. Conjugate as in any of claims 24 to 26, **characterized in that** the molecule or molecules having on their surface at least one free thiol function before conjugation are markers.

28. Conjugate as in claim 27, **characterized in that** the markers are alkaline phosphatase previously modified to include free thiol functions.

29. Use of a conjugate as in any of claims 24 to 28 for *in vitro* diagnostic methods of diseases involving recognition of a ligand-anti-ligand pair.

## Patentansprüche

1. Kupplungsmittel der folgenden allgemeinen Formel: 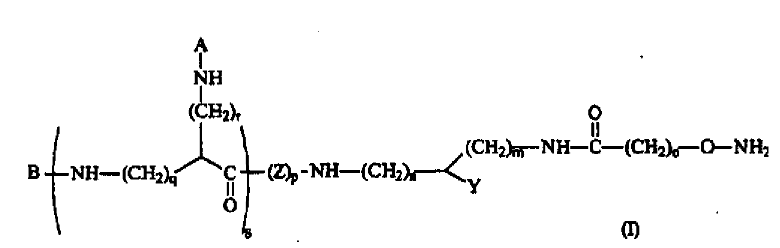 wobei:
- Z für -NH-(CH₂)₂-O-(CH₂)₂-O-CH₂-C(O)- steht
- A für eine Gruppe steht, ausgewählt aus: 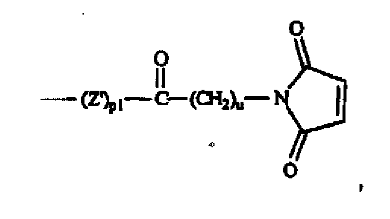 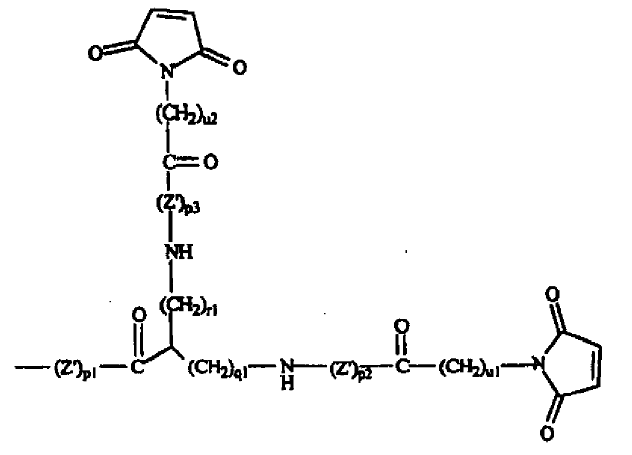 und 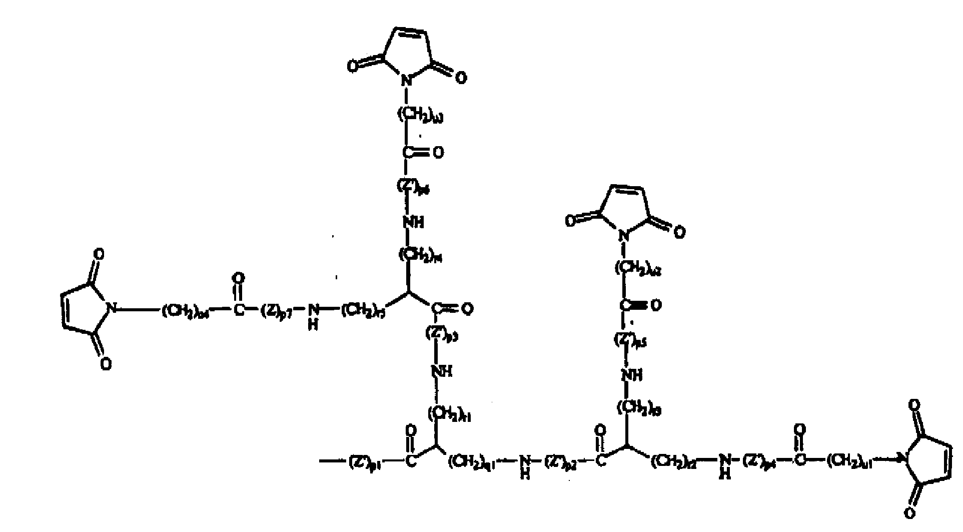 B für eine Gruppe steht, ausgewählt aus: 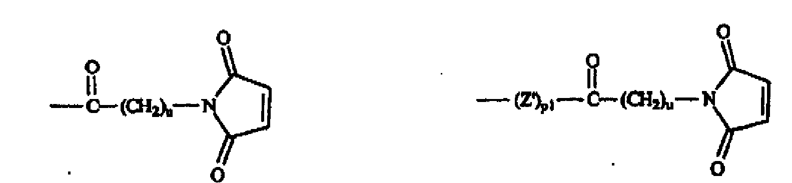 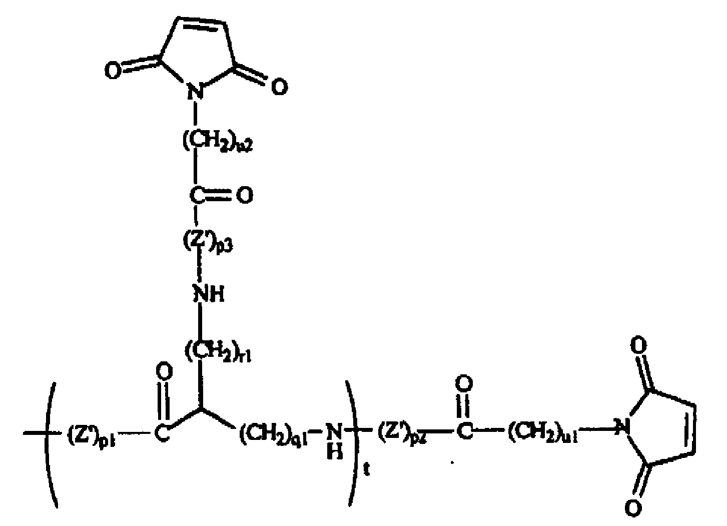 und 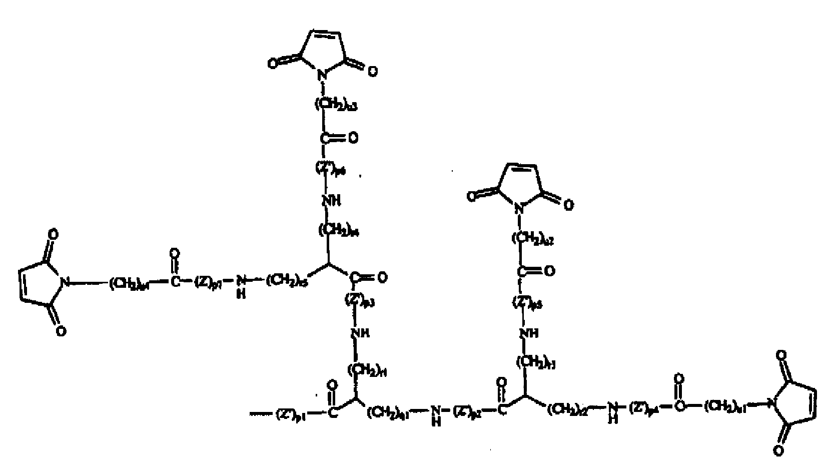
- Y für eine Gruppe steht, die mit -C(O)-NH₂ endet und die gegenüber der Aminooxyalkylenfunktion, der oder den Maleimidgruppen von B und gegebenenfalls von A sowie dem Motiv -(CH₂)₂-O-(CH₂)₂-O-CH₂- von Z inert ist,
- Z' für -C(O)-CH₂-O-(CH₂)₂-O-(CH₂)₂-NH- steht,
- n eine ganze Zahl im Bereich von 0 bis 3 ist,
- m eine ganze Zahl im Bereich von 1 bis 3 ist,
- o eine ganze Zahl im Bereich von 1 bis 3 ist,
- p und p1 bis p7 jeweils unabhängig voneinander eine ganze Zahl im Bereich von 1 bis 4 sind,
- q und q1 jeweils unabhängig voneinander eine ganze Zahl im Bereich von 0 bis 3 sind,
- r und r1 bis r5 jeweils unabhängig voneinander eine ganze Zahl im Bereich von 2 bis 5 sind,
- s eine ganze Zahl ist, die gleich 0 oder 1 ist,
- t eine ganze Zahl im Bereich von 1 bis 7 ist, und
- u und u1 bis u4 jeweils unabhängig voneinander eine ganze Zahl im Bereich von 2 bis 10 sind.

2. Kupplungsmittel nach Anspruch 1, **dadurch gekennzeichnet, daß** Y -C(O)-NH₂ ist.

3. Kupplungsmittel nach einem der Ansprüche 1 oder 2, **dadurch gekennzeichnet, daß** s gleich 0 ist.

4. Kupplungsmittel nach Anspruch 3, **dadurch gekennzeichnet, daß** B steht für: 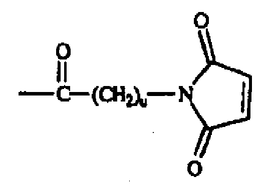 wobei u der Begriffsbestimmung in Anspruch 1 entspricht.

5. Kupplungsmittel nach einem der Ansprüche 1 oder 2, **dadurch gekennzeichnet, daß** A und B stehen für: 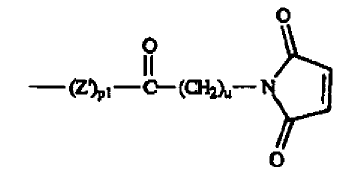 wobei Z', p1 und u den Begriffsbestimmungen in Anspruch 1 entsprechen.

6. Kupplungsmittel nach einem der Ansprüche 1 oder 2, **dadurch gekennzeichnet, daß** A und B stehen für: 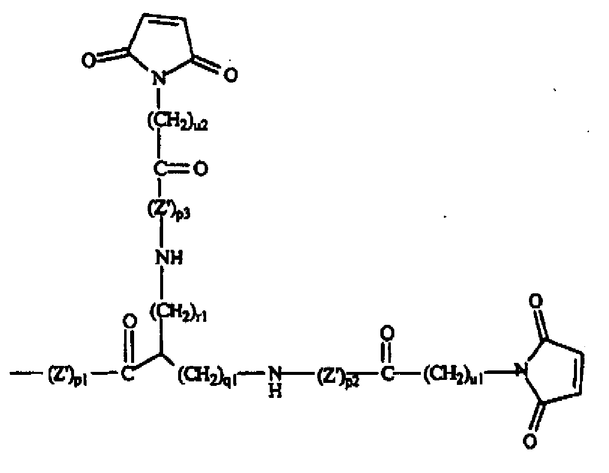 wobei Z', p1, p2, p3, q1, r1, u1 und u2 den Begriffsbestimmungen in Anspruch 1 entsprechen.

7. Kupplungsmittel nach einem der Ansprüche 1 oder 2, **dadurch gekennzeichnet, daß** A und B stehen für: 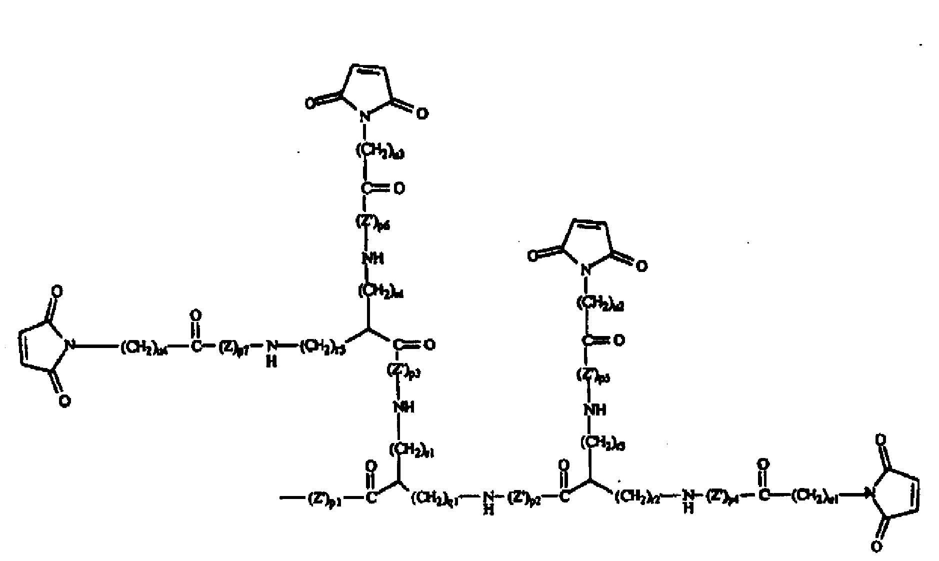 wobei Z, Z', p1 bis p7, q1, r1 bis r5 und u1 bis u4 den Begriffsbestimmungen in Anspruch 1 entsprechen.

8. Kupplungsmittel nach einem der Ansprüche 1 oder 2, **dadurch gekennzeichnet, daß** A steht für: 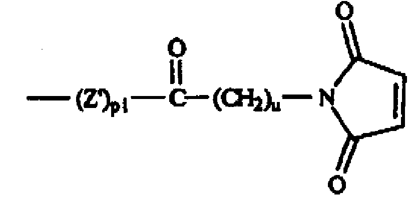 und
B steht für: 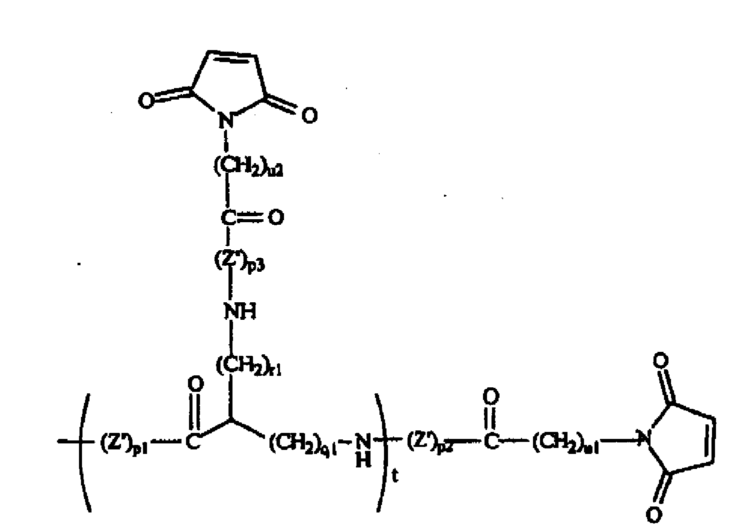 wobei Z', t, p1 bis p3, q1. r1, u, u1 und u2 den Begriffsbestimmungen in Anspruch 1 entsprechen.

9. Kupplungsmittel nach einem beliebigen der vorhergehenden Ansprüche, **dadurch gekennzeichnet, daß** n gleich 0 ist.

10. Kupplungsmittel nach einem beliebigen der vorhergehenden Ansprüche, **dadurch gekennzeichnet, daß** m gleich 1 ist.

11. Kupplungsmittel nach einem beliebigen der vorhergehenden Ansprüche, **dadurch gekennzeichnet, daß** p und p1 bis p7 gegebenenfalls gleich 2 sind.

12. Kupplungsmittel nach einem beliebigen der vorhergehenden Ansprüche, **dadurch gekennzeichnet, daß** q und q1 gegebenenfalls gleich 0 sind.

13. Kupplungsmittel nach einem beliebigen der vorhergehenden Ansprüche, **dadurch gekennzeichnet, daß** r und r1 bis r5 gegebenenfalls gleich 4 sind.

14. Kupplungsmittel nach einem beliebigen der vorhergehenden Ansprüche, **dadurch gekennzeichnet, daß** u und u1 bis u4 gegebenenfalls gleich 2 sind.

15. Kupplungsmittel nach einem beliebigen der vorhergehenden Ansprüche, **dadurch gekennzeichnet, daß** o gleich 1 ist.

16. Kupplungsmittel nach Anspruch 1, **dadurch gekennzeichnet, daß** es sich dabei um Folgendes handelt:
- die Verbindung nach Formel (I), wobei m = 1, Y = -C(O)-NH₂. o = 1, n = 0, p = 2, s = 0 und B steht für: 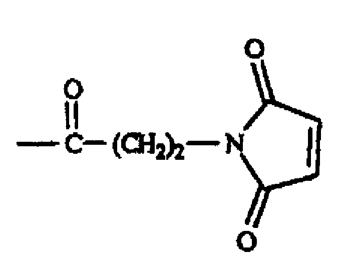 oder
- die Verbindung nach Formel (I), wobei m = 1, Y = -C(O)-NH₂, o = 1, n = 0, p = 2, r = 4, s = 1, q = 0 und A und B identisch sind und stehen für: 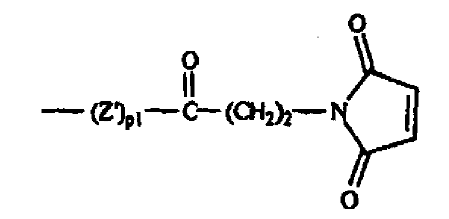 wobei p1 gleich 2 ist.

17. Aktivierte Zwischenverbindung, die aus einem Kupplungsmittel nach einem beliebigen der Ansprüche 1 bis 16 und aus einem Molekül besteht, das vor der Konjugation an seiner Oberfläche mindestens eine Aldehyd- und/oder Ketonfunktion aufweist.

18. Aktivierte Zwischenverbindung nach Anspruch 17, **dadurch gekennzeichnet, daß** das Molekül, welches an seiner Oberfläche mindestens eine Aldehydfunktion aufweist, ein mittels Periodat oxydiertes Glycoprotein ist.

19. Aktivierte Zwischenverbindung nach Anspruch 18, **dadurch gekennzeichnet, daß** es sich bei dem Glycoprotein um das Glycoprotein gp160 handelt.

20. Aktivierte Zwischenverbindung, die aus einem Kupplungsmittel nach einem beliebigen der Ansprüche 1 bis 16 und aus einem bis acht Molekülen besteht, die vor der Konjugation an ihrer Oberfläche mindestens eine freie Thiolfunktion aufweisen.

21. Aktivierte Zwischenverbindung nach Anspruch 20, **dadurch gekennzeichnet, daß** sie ein bis vier Moleküle enthält, die vor der Konjugation an ihrer Oberfläche mindestens eine freie Thiolfunktion aufweisen.

22. Aktivierte Zwischenverbindung nach einem der Ansprüche 20 oder 21, **dadurch gekennzeichnet, daß** es sich bei dem oder den anderen Molekül(en), das/die an seiner/ihrer Oberfläche mindestens eine freie Thiolfunktion aufweist/aufweisen, um einen Marker handelt.

23. Aktivierte Zwischenverbindung nach Anspruch 22, **dadurch gekennzeichnet, daß** es sich bei dem Marker um alkalische Phosphatase handelt, die zuvor derart modifiziert wurde, daß sie freie Thiolfunktionen umfasst.

24. Konjugat, das aus einem Kupplungsmittel nach einem beliebigen der Ansprüche 1 bis 16 besteht, welches an ein Molekül, das vor der Konjugation an seiner Oberfläche mindestens eine Aldehyd- und/oder Ketonfunktion aufweist, und an ein bis acht Moleküle, die vor der Konjugation an ihrer Oberfläche mindestens eine freie Thiolfunktion aufweisen, gebunden ist.

25. Konjugat nach Anspruch 24, **dadurch gekennzeichnet, daß** es sich bei dem Molekül, welches vor der Konjugation an seiner Oberfläche mindestens eine Aldehyd- und/oder Ketonfunktion aufweist, um ein Molekül von biologischem Interesse handelt.

26. Konjugat nach Anspruch 25, **dadurch gekennzeichnet, daß** es sich bei dem Molekül von Interesse um das zuvor oxidierte Glycoprotein gp160 handelt.

27. Konjugat nach einem beliebigen der Ansprüche 24 bis 26, **dadurch gekennzeichnet, daß** es sich bei dem oder den Moleküle(n), das/die an seiner/ihrer Oberfläche mindestens eine freie Thiolfunktion aufweist/aufweisen, um Marker handelt.

28. Konjugat nach Anspruch 27, **dadurch gekennzeichnet, daß** es sich bei den Markern um alkalische Phosphatase handelt, die zuvor derart modifiziert wurde, daß sie freie Thiolfunktionen umfasst.

29. Verwendung eines Konjugats nach einem beliebigen der Ansprüche 24 bis 28 in Rahmen von in-vitro-Diagnoseverfahren für Krankheiten, welche eine Erkennung eines Ligand-Antiligand-Paares beinhalten.
